# EUROPEAN PATENT SPECIFICATION

(11) **EP 2 680 771 B1**
(45) Date of publication and mention of the grant of the patent: **19.06.2019**
(21) Application number: 12753011.1
(22) Date of filing: 28.02.2012
(51) Int. Cl.: A61K 38/18, A61K 38/30, A61F 2/44, A61F 2/28, A61F 2/30, A61B 17/70

(54) **DEGRADABLE CAGE FOR BONE FUSION**
ABBAUBARER KÄFIG ZUR KNOCHENVERBINDUNG
CAGE DÉGRADABLE POUR FUSION OSSEUSE

(30) Priority: 28.02.2011 US 201113036470
(43) Date of publication of application: 08.01.2014
(73) Proprietor: Tissue Regeneration Systems, Inc., Ann Arbor, MI 48104 (US)
(72) Inventor: LIN, Chia-Ying, Ann Arbor, Michigan 48108 (US); LAMARCA, Frank, Ann Arbor, Michigan 48104 (US); HOLLISTER, Scott J., Saline, Michigan 48176 (US)
(74) Representative: Klunker IP Patentanwälte PartG mbB
(86) International application number: PCT/US2012/027000
(87) International publication number: WO 2012/118846

(56) References cited:
- US-A- 5 458 642
- US-A1- 2003 208 274
- US-A1- 2008 215 093
- US-B2- 7 101 400

## Description

### RELATED APPLICATIONS

The present application claims the benefit of priority to U.S. Nonprovisional Application Serial No. 13/036,470 filed 28 February 2011; which is a continuation-in-part of U.S. Nonprovisional Application Serial No. 11/927,322 filed 29 October 2007; which claims the benefit of U.S. Provisional Application Serial No. 60/855,235, filed 30 October 2006.

### BACKGROUND OF THE INVENTION

### Field of the Invention

This invention relates to cages for facilitating the fusion of adjacent bones or adjacent bone surfaces, and more particularly to degradable cages for spinal interbody fusion.

### Description of the Related Art

Back pain resulting from instability of the spinal system is a rapidly growing condition in the United States. Spinal fusion procedures are expected to grow from over 400,000 procedures in 2004 to 550,000 procedures in 2010. This is driven by an aging population, increasing obesity, and increased patient education and awareness of the fusion procedures. While current segmental spinal fusion relieves pain by eliminating spinal instability, complications associated with conventional metallic cages, including; difficulty of revisions, increased adjacent level disc disease due to increased loading, implant migration or failure, imaging artifacts, stress shielding, and limited bone grafting significantly reduce the efficacy of the interbody fusion. Non-degradable polymeric materials such as polyetheretherketone (PEEK) have been introduced as new cage materials as they are radiotransparent and compliant and can enhance post-operative image modality and fusion rate. However, since clinically reliable reports of using these cages are scarce, concerns still remain that synovitis and the lymphatic spread of non-absorbable polymer debris may be found after intra-articular procedures (see Cho et al., "Preliminary experience using a polyetheretherketone (PEEK) cage in the treatment of cervical disc disease" Neurosurgery 52(3):693 2003 and Neurosurgery 51:1343 2002; and Parsons et al., "Carbon fiber debris within the synovial joint. A time-dependent mechanical and histologic study", Clinical Orthopaedics & Related Research 1985:69-76).

It has been reported that spine musculoskeletal impairments, including degenerative disc disease, stenosis, spondylolysis, and/or spondylolisthesis, represent more than one-half (51.7% or 15.4 million incidents) of the musculoskeletal impairments reported in the United States. In the United States, 279,000 spinal arthrodesis were performed in 1990, with 26 lumbar fusions performed per 100,000 people (see Andersson, "Epidemiological features of chronic low-back pain", Lancet 354:581-5, 1999). In 1995, approximately 160,000 spinal fusion surgeries were performed (see Praemer et al. "Musculoskeletal Conditions in the United States" Park Ridge: American Academy of Orthopaedic Surgeons, 1999). A recent report in 2000 (see Sanhu, "Anterior lumbar interbody fusion with osteoinductive growth factors", Clinical Orthopaedics and Related Research 371:56-60, 2000) revealed that in the United States alone approximately 360,000 patients underwent some type of spinal arthrodesis. The use of cage devices has become an adjunct to interbody fusion for degenerative disorders of the lumbar spine. However, current metallic cages are associated with excessive rigidity that increases incidence of postoperative complications such as stress-shielding, the migration or dislodgement of the cage, pseudoarthrosis, or the combined adverse symptoms (see van Dijk et al. "The effect of cage stiffness on the rate of lumbar interbody fusion: An in vivo model using poly(L-lactic acid) and titanium cages", Spine 27:682-8, 2002). Metallic cages can also interfere with visual assessment of arthrodesis and the integrity of the spinal canal and neural foramina due to image artifact. The stress-shielded environment resulting from excessive metallic cage stiffness lowers intracage pressure (see Kanayama et al., "In vitro biomechanical investigation of the stability and stress-shielding effect of lumbar interbody fusion devices", Journal of Neurosurgery 93:259-65, 2000), leading to subsequent decreased mineralization, bone resorption, and significant bone mineral density decrease in long-term (see Cunningham et al., "A quantitative densitometric study investigating the stress-shielding effects of interbody spinal fusion devices: Emphasis on long-term fusions in thoroughbred racehorses", Trans Orthop Res Soc 23:250, 1998).

Many current efforts to reduce these complications have concentrated on using poly (a-hydroxy) ester polymers that have much lower stiffness than metallic materials to fabricate conventional cage designs (see, Kandziora et al., "Biomechanical analysis of biodegradable interbody fusion cages augmented with poly(propylene glycol-co-fumaric acid)", Spine 27:1644-51, 2002; Toth et al., "Evaluation of 70/30 poly (L-lactide-co-D,L-lactide) for use as a resorbable interbody fusion cage", Journal of Neurosurgery 97:423-32, 2002; van Dijk et al., "Bioabsorbable poly-L-lactic acid cages for lumbar interbody fusion: three-year follow-up radiographic, histologic, and histomorphometric analysis in goats", Spine 27:2706-14, 2002). Degradable cages possess a number of significant advantages over non-degradable materials including eventual removal of all foreign material that could cause nerve root irritation, alleviation of stress-shielding effects and reduce adjacent level disc disease, and removal of imaging artifact. Nevertheless, the mere replacement of base material from original designs might lead to cages that cannot provide adequate stability since biodegradable polymers have less stiffness/strength than permanent materials and this reduced stiffness/strength will be further compromised over the degradation time. Furthermore, primary degradation products of these poly (a-hydroxy) acids form a low pH environment that can inhibit osteogenesis. It has been shown that even small pH shifts can significantly affect bone marrow stromal cell (BMSC) function of proliferation and differentiation (see Kohn et al., "Effects of pH on human bone marrow stromal cells in vitro: Implications for tissue engineering of bone", Journal of Biomedical Materials Research 60:292-9, 2002) since the growth and development of osteoblasts are linked to regulation of pH and acidity of the extracellular microenvironment (see Chakkalakal et al., "Mineralization and pH relationships in healing skeletal defects grafted with demineralized bone matrix" Journal of Biomedical Materials Research 28:1439-43, 1994; Green "Cytosolic pH regulation in osteoblasts", Mineral and Electrolyte Metabolism 20:16-30, 1994; Kaysinger et al., "Extracellular pH modulates the activity of cultured human osteoblasts", Journal of Cellular Biochemistry 68:83-913-15, 1998). Therefore, although degradable polymer cages offer significant potential advantages over non-degradable cages, there are also significant hurdles to overcome including the maintenance of adequate mechanical properties and reduction of acidic degradation products.

With various bone graft substitutes emerging as biological inducers to achieve successful arthrodesis, delivery within a restricted volume becomes critical. Among a variety of promising bone graft substitutes, bone growth factors and cell-based approaches particularly require suitable delivering vehicles (see Helm et al. "Bone graft substitutes for the promotion of spinal arthrodesis", Neurosurg Focus 10:1-5, 2000). Several recombinant human bone morphogenic proteins (rh-BMPs) have been approved for certain clinical applications, and they are commonly delivered through an absorbable collagen sponge to effectively achieve arthrodesis by osteoinduction. However, current delivering approaches are associated with the inability to directly deliver bone morphogenic proteins for bone regeneration.

The only commercially available delivery system at this moment for bone morphogenic protein consists of collagen sponges soaked in bone morphogenic protein solutions that contain bone morphogenic protein at concentrations over a million times higher than what is physiologically found in the human body. The release of the bone morphogenic protein in this fashion obviously consists of a very large bolus quantity of which all its effects are unknown. Reports have shown that bone morphogenic protein can also cause an initial osteolysis of surrounding bone secondary to what is thought to be an initial drain of osteogenic cells from surrounding bone towards the bone morphogenic protein soaked sponges. This can initially weaken surrounding bone structures thus promoting subsidence of any supporting implants. Furthermore, a high concentration of bone morphogenic protein has been shown to cause swelling of surrounding soft tissue with resultant swallowing and breathing difficulty. Another disadvantage of uncontrolled release of bone morphogenic protein is the ectopic formation of bone. Bone formation distal from the intended site of osteogenesis can result in radiculopathy as well as intradural bone formation.

Primary requirements in developing biodegradable cages are assuring that porous degradable cages can withstand surgical impaction forces, can carry in vivo spinal forces initially and up to the time bony fusion is achieved (normally 3-6 months), and have degradation products that will not adversely affect bone regeneration. However, bone tissue engineering within degradable constructs invokes two new requirements in addition to the primary degradable cage requirements delineated above. The first is osteoconductivity, which is the ability to promote and support ingrowth of bone-forming cells. Among the most common strategies to confer osteoconductivity to an orthopedic implant material involves coating with a calcium-phosphate-based mineral film similar to bone mineral. These films have a well-characterized positive effect on the ingrowth and proper function of bone-forming cell types, including osteoblasts and osteoblast precursors. U.S. Patent No. 6,767,928 shows that calcium-phosphate mineral coatings can be grown on porous polymer scaffolds, and that the mineral coatings positively influence bone tissue growth. The technology used to grow these mineral coatings mimics the process of natural bone mineralization, and the coatings have a structure, mineral phase, and elemental composition that is similar to human bone mineral (see also, Bunker et al., "Ceramic thin film formation on functionalized interfaces through biomimetic processing", Science 264:48-55, 1994; Mann et al., "Crystallization and inorganic-organic interfaces - biominerals and biomimetic synthesis", Science 261:1286-92, 1993; Murphy et al., "Bioinspired growth of crystalline carbonate apatite on biodegradable polymer substrata", J Am Chem Soc 124:1910-7, 2002; and Ohgushi et al., "Stem cell technology and bioceramics: from cell to gene engineering", J Biomed Mater Res 48:913-27, 1999). These "bone-like" mineral coatings have been shown to significantly enhance osteoconductivity of orthopedic implant materials (see Ohgushi et al.; Hench, "Bioceramics: From concept to clinic", Journal of the American Ceramic Society 74: 1487 -510, 1991; Murphy et al., "Bone regeneration via a mineral substrate and induced angiogenesis", J Dent Res 83:204-10,2004). In addition to their osteoconductivity, mineral coatings also represent a potential vehicle for delivery of osteogenic growth factors (see Seeherman et al., "Bone morphogenetic protein delivery systems", Spine 27:S16-23, 2002). Multiple bone growth factors, including BMP-2, IGF-1 and TGF-13 (see Gittens et al., "Imparting bone mineral affinity to osteogenic proteins through heparin-bisphosphonate conjugates", J Control Release 98:255-68, 2004; Gorski et al., "Is all bone the same? Distinctive distributions and properties of non-collagenous matrix proteins in lamellar vs. woven bone imply the existence of different underlying osteogenic mechanisms", Crit Rev Oral Bioi Med 9:201-23, 1998; Gorski et al., "Bone acidic glycoprotein-75 is a major synthetic product of osteoblastic cells and localized as 75- and/or 50-kDa forms in mineralized phases of bone and growth plate and in serum", J Bioi Chem 265:14956-63, 1990; Liu et al., "Bone morphogenetic protein 2 incorporated into biomimetic coatings retains its biological activity", Tissue Eng 10:101-8, 2004; Matsumoto et al., "Hydroxyapatite particles as a controlled release carrier of protein", Biomaterials 25:3807-12,2004; and Sachse et al., "Osteointegration of hydroxyapatite-titanium implants coated with nonglycosylated recombinant human bone morphogenetic protein-2 (BMP2) in aged sheep", Bone 37:699-710, 2005) have been shown to interact strongly with bone-like mineral substrates. Therefore, it is possible that calcium phosphate mineral substrates can be coated with growth factors, and these factors can subsequently be presented to bone-forming cells growing into a scaffold construct. Previous studies have demonstrated that it is indeed possible to use hydroxyapatite minerals as template substrates to bind and release bone growth factors, particularly BMP2, and that the bound growth factors induce bone ingrowth *in vivo* (see Gittens *et al.;* and Sachse *et al*.).

US Patent Publication No, 2008/0215093 entitled "Degradable Cage Coated with Mineral Layers for Spinal Interbody Fusion" describes a cage for facilitating fusion of bones, that may include a bioactive agent to induce ossification between bones. Notwithstanding the foregoing advances in tissue engineering, there is still a need for improved cages for facilitating the fusion of adjacent bones such as vertebrae, or adjacent bone surfaces such as in an open fracture.

### SUMMARY OF THE INVENTION

The inventors have developed the necessary design and fabrication techniques to create optimized degradable spine fusion cages to fulfill the load carrying requirement and have also created prototype cages from a biodegradable polymer polycaprolactone (PCL) using Solid Free-Form Fabrication (SFF) techniques. This pilot work has helped characterize the designed biodegradable cages and define the most appropriate design for incorporating with therapeutic bioactive agents to facilitate spinal arthrodesis.

The structurally tailored design that is able to fulfill the mechanical load bearing requirements is incorporated with innovative mineralization processes to enhance the bioactivity of the spinal implant. The mineral coated PCL cage is also believed to have superior binding capacity and persistent delivery of therapeutic molecules such as bone morphogenetic proteins (BMP) compared to current approaches such as collagen sponges.

The invention relates to a cage for facilitating fusion of adjacent bone segments. The cage comprises:
a designed porous microstructure comprising a biocompatible material; and
a fixation plate comprising a central portion, wherein the cage further comprises a plurality of substantially parallel spaced apart walls, the walls interconnected by transverse projections; the walls are substantially parallel to the central portion of the fixation plate and are coupled to the central portion of the fixation plate through at least one support, the support having a proximal end and a distal end, the support being joined at the proximal end to the central portion of the fixation plate, the support extending perpendicularly from the central portion.

In addition, a method of fusing two bone segments is described herein. The method comprises inserting the above cage between the two bone segments such that substantially all of the parallel spaced apart walls abut the two bone segments.

These and other features, aspects, and advantages of the present invention will become better understood upon consideration of the following detailed description, drawings and appended claims.

### BRIEF DESCRIPTION OF THE DRAWINGS

FIG. 1 shows a cervical spine fusion cage design with an integrated anterior fixation plate and designed microstructure. Panel A shows a right rear perspective view of the design. Panel B shows a top front left perspective view of the design.
FIG. 2 shows a bottom perspective view of the design of FIG. 1.
FIG. 3 shows the results of coating polycaprolactone with calcium phosphate. In particular, FIG. 3 shows scanning electron microscope (SEM) micrographs showing large scale pore structure of polycaprolactone scaffolds without calcium phosphate coating (left, top right), and with calcium phosphate coating (bottom right).
FIG. 4 shows an X-ray diffraction spectrum showing hydroxyapatite (^{*}) grown on a polycaprolactone (^{∧}) scaffold. The inset is a high magnification SEM image of a calcium phosphate coating grown on a polycaprolactone scaffold.

FIG. 5 shows a top front left perspective view of a lumbar spine fusion cage design with an integrated lateral plate for fixation.

FIG. 6 shows a cervical spine fusion cage design according to an embodiment of the invention. Panel A shows a perspective of the design. Panel B shows a photograph of a cage prepared using said design.

FIG. 7 shows the cage design of FIG. 1, as scaled for implantation into a Yucatan minipig. Panel A is an isometric view showing the designed interbody region (5.3 mm height). Panel B is an inferior-superior view showing Anterior-Posterior (AP) and Medial-Lateral (ML) dimensions.

FIG. 8 shows a manufactured prototype of the cage designed as in FIG. 7. Panel A shows an Inferior-Superior view. Panel B shows an Anterior-Posterior view.

FIG. 9 shows a micro-CT scan of a cage as shown in FIG. 8. The scan demonstrated the fidelity of manufactured device to design with extremely low defect volume, less than 0.6%.

FIG. 10 is photographs and micrographs of aspects of calcium phosphate (CaP) coating of a cage as shown in FIG. 6. Panel A shows the coated cage. Panels B-D are scanning electron micrographs (SEM) of (B) uncoated surface; (C) coated surface showing the CaP coating and changes in surface morphology (scale bar 500 µm); and (D) high magnification of the CaP coating (scale bar 2 µm).

FIG. 11 shows an analysis of the cage CaP coating composition using energy dispersive spectroscopy (EDS). Panel A shows EDS results from an uncoated area of the cage, exhibiting minimum Ca and P. Panels B and C show EDS results from coated areas cages, exhibiting the presence of significant Ca and P in the coating, in a ratio of 1.1 to 1.3. Coating characteristics are also shown in the SEM image (Panel D)(scale bar 100 µm) and X-ray diffraction patterns (Panel E).

FIG. 12 shows surgical implantation of integrated plate/cage device in Yucatan minipig. Panel A shows the anterior approach. Panel B shows the cage placement. Panel C shows the fixed cage. Panel D shows the cage after affixing with screws.

FIG. 13 shows CT scans from 1.5 to 9 months after implanting a polycaprolactone/calcium phosphate (PCL/CaP) coated cage in a Yucatan minipig from Group 1 of the animal study described in the Example. Bony bridging in the interbody device (circles) progresses to fusion by 9 months.

FIG. 14 shows a micro-CT scan slice of a PCL/CaP cage at 18 months. Complete bone growth is seen through the cage regions (box) demonstrating fused vertebrate. Holes outside of box are screw holes.

FIG. 15 is a graph showing percent bone fill in the three treatment groups of the study described in the Example, measured 6, 12, and 18 months after implantation. All groups demonstrated increasing bone fill with time. The PCL/CaP coated device without biologics ("8 day min") demonstrated similar levels of bone fill as an uncoated device delivering BMP7 protein.

Like reference numerals will be used to refer to like or similar parts from Figure to Figure in the following description.

### DETAILED DESCRIPTION OF THE INVENTION

One purpose of the proposed invention is to develop a simple and flexible method that enhances osteogenesis to achieve spine arthrodesis induced by biologically active bone morphogenetic proteins released from osteoconductive, biodegradable spine fusion cages. The Example describes experiments where a polycaprolactone cage was implanted into the a cervical intervertebral space of a Yucatan minipig model. In those experiments, some bone formation was achieved in a scaffold without a bioactive agent or a calcium phosphate mineral coating, but a bioactive agent (here, BMP7 in a collagen sponge) and mineral coating enhances bone formation. The calcium phosphate mineral coatings was deposited on the polycaprolactone cages using a low temperature process. Bioactive agents can be incorporated into the mineral coating, *e.g.,* via surface binding. See PCT Patent Application PCT/US09/58419, entitled MINERAL-COATED MICROSPHERES, filed September 25, 2009. The resulting composite cage contains biologically active growth factors, which are released upon mineral dissolution and/or degradation of the cage.

The invention of the designed degradable interbody fusion system may represent a transition from passive support of bone graft material within the intervertebral space (*e.g.,* traditional dense cage designs) to a more aggressive strategy of spinal tissue engineering.

### Interbody Fusion Cage Design

U.S. Patent Application Publication No. 2003/0069718 (which corresponds to U.S. Patent No. 7,174,282) provides a nonlimiting example of a design methodology for creating biomaterial scaffolds with internal porous architectures that meet the need for mechanical stiffness and strength and the need for connected porosity for cell migration and tissue regeneration. See also U.S. Patent Application Publication No. 2008/0195211, and Provisional Patent Application Serial No. entitled MODULAR SCAFFOLDS AND IMPLANTS, filed concurrently with this application. The design methods of U.S. 2003/0069718 combine image-based design of structures with homogenization theory to compute effective physical property dependence on material microstructure. Optimization techniques are then used to compute the optimal geometry. The final optimized scaffold geometry voxel topology is then combined with a voxel data set describing the three dimensional anatomic scaffold shape which may be obtained by magnetic resonance (MR) images or combined MR and computed tomography (CT) images. Density variations within the anatomic scaffold voxel database are used as a map to guide where different optimized scaffold voxel topologies are substituted. The final voxel representation of the anatomically shaped scaffold with optimized interior architecture is then converted automatically by software into either a surface representation or wire frame representation for fabrication of the scaffold by way of solid free form fabrication or casting.

The interbody fusion cages exemplified herein were designed based on a CT scan of a cadaver Yucatan minipig cervical spine. The integrated topology optimization technique was utilized to create a cage design based on the techniques of U.S. 2003/0069718. An optimization program can be run to predict densities at different time points in the degradation profile, thus incorporating degradation into the design. In the degradation design, the density in each element is weighted by the degradation profile. The optimization method creates a density distribution map for selected time points during degradation. These different density distributions are then superposed using a time lasting and degrading modulus factor. The time lasting factor: defined as T_{wt} = (Tₜₒₜₐₗ - T_{current})/Tₜₒₜₐₗ, where Tₜₒₜₐₗ is total degradation duration, T_{current} is the time at a selected point. This factor accounts for the influence of the time past implantation on reinforcement of the scaffold architecture. The degrading modulus factor is defined as E_{wt} = E⁰ᵢⱼₖₗ(T_{current})/ E⁰ᵢⱼₖₗ(Tᵢₙᵢₜᵢₐₗ). The factor indicates the weight percentage of the original material equivalent to the superposed material densities based on the degrading modulus at selected time points. The optimal global/macroscopic density distribution for degradation design is then interpreted into X_{pw} = XₚₜT_{wt}E_{wt}, where X_{pw} is the final fraction of the base material, and Xₚₜ is the temporary fraction of the reduced/degraded modulus corresponding to a selected time point. The approach creates cages designed to retain desired stiffness after a specified degradation period.

The resolution of the global degradation topology design is too coarse, however, to give the specific microstructure that will be located within that point of the scaffold. Furthermore, since the microstructure is desired to have specific elastic properties at a fixed porosity, homogenization based topology optimization is used to design the microstructure (see Hollister et al. "Optimal design and fabrication of scaffolds to mimic tissue properties and satisfy biological constraints", Biomaterials 23:4095-103, 2002; and Lin et al. "A novel method for internal architecture design to match bone elastic properties with desired porosity", Journal of Biomechanics 37:623-36, 2004). The microscopic or 2nd scale topology optimization approach gives the specific microstructure design that achieves a desired compliance while matching the predicted volume fraction of the macroscopic or 1st level topology optimization.

In the interior cage microstructure design, the image-based methods as in U.S. 2003/0069718 can be used to design an interior cage with internal architecture optimized to match target bone Young's moduli. In particular, the minimum and maximum interior cage Young's moduli could be set to 1 and 15 GPa, respectively, to reflect the Young's modulus of available scaffold material ranging from biopolymers (E = 1 GPa) to bioceramics (E = 15 GPa). This can optimize strain for bone growth. Also, the modulus ranges for trabecular bone and intevertebral disc that we want to target for fusion and disc repair are: Bone: 30 -200 MPa, and Intervertebral Disc: 0.4 -10 MPa.

In addition to the interior cage microstructure design, the fixation structure for the cage can also be designed using image-based methods as in US 2003/0069718.

Using the above design methods, cages for facilitating fusion of adjacent bone segments were designed. The cage comprises: a designed porous microstructure comprising a biocompatible material; a plurality of substantially parallel spaced apart walls, the walls interconnected by transverse projections; and a fixation plate comprising a central portion. In these cages, the walls are coupled to the central portion of the fixation plate.

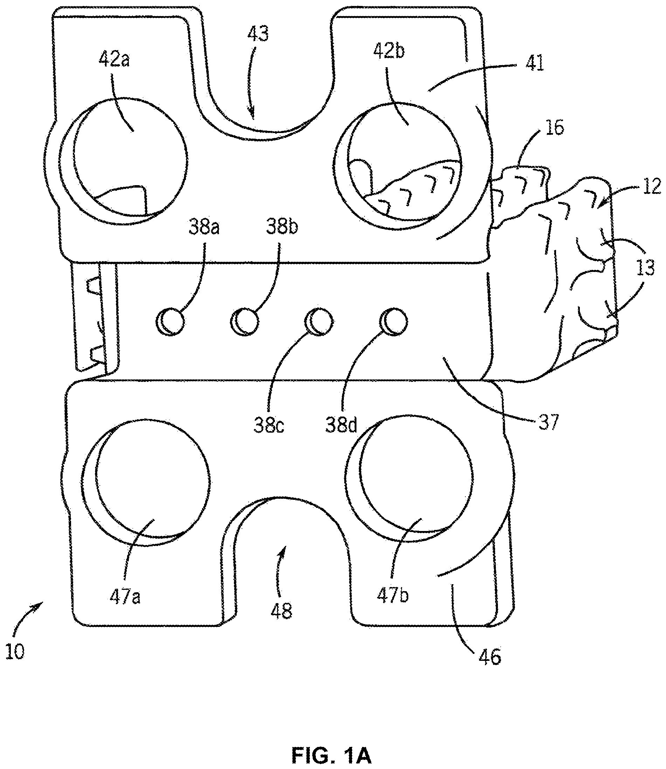

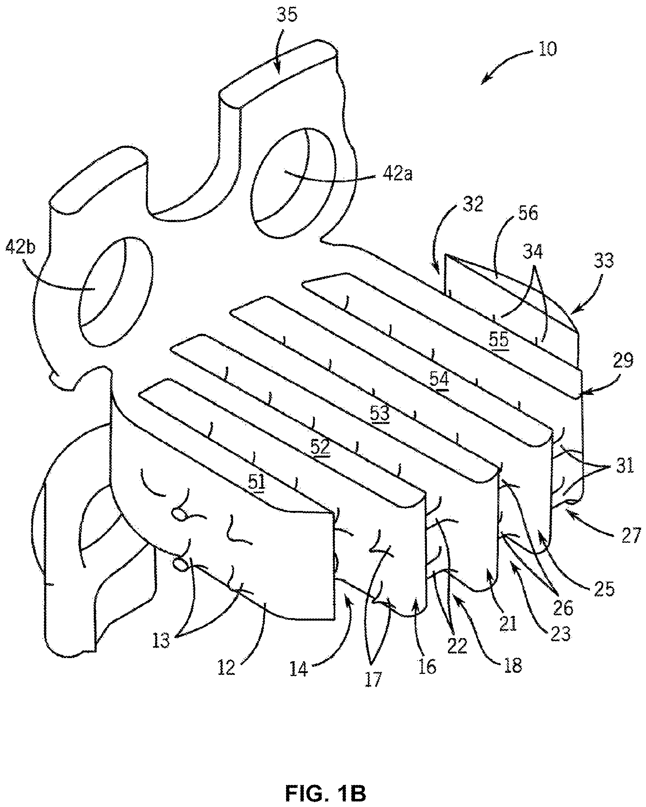

One example of this design is shown in FIGS. 1A and 1B, providing illustrations of a cervical spine fusion cage 10, that is integrated with anterior plate fixation.

The cage 10 has parallel spaced apart walls 12, 16, 21, 25, 29, 33 that are substantially perpendicular to the central portion of the fixation plate 37. Specifically provided is a first vertical wall 12 having a substantially rectangular transverse vertical cross section. The first wall 12 has projections 13 extending substantially perpendicularly from a vertical side surface of the first wall 12. A first space 14 is created between the first wall 12 and a second vertical wall 16 having a substantially rectangular transverse vertical cross section. The second wall 16 has projections 17 extending substantially perpendicularly from a vertical side surface of the second wall 16. A second space 18 is created between the second wall 16 and a third vertical wall 21 having a substantially rectangular transverse vertical cross section. The third wall 21 has projections 22 extending substantially perpendicularly from a vertical side surface of the third wall 21. A third space 23 is created between the third wall 21 and a fourth vertical wall 25 having a substantially rectangular transverse vertical cross section. The fourth wall 25 has projections 26 extending substantially perpendicularly from a vertical side surface of the fourth wall 25. A fourth space 27 is created between the fourth wall 25 and a fifth vertical wall 29 having a substantially rectangular transverse vertical cross section. The fifth wall 29 has projections 31 extending substantially perpendicularly from a vertical side surface of the fifth wall 29. A fifth space 32 is created between the fifth wall 29 and a sixth vertical wall 33 having a substantially rectangular transverse vertical cross section. The sixth wall 33 has projections 34 extending substantially perpendicularly from a vertical side surface of the sixth wall 33. It is to be understood that, although the cage exemplified in FIG. 1 shows six walls, the cages provided herein can have two, three, four, five, seven, eight or more walls.

Still referring to FIGS. 1A and 1B, the cage 10 has a fixation plate 35 having a central section (i.e., a central portion) 37 with throughholes 38a, 38b, 38c, 38d. The walls 12, 16, 21, 25, 29, 33 are integral with the central portion 37 of the fixation plate 35. The walls 12, 16, 21, 25, 29, 33 are substantially perpendicular to the fixation plate 35. The fixation plate 35 includes a top section 41 that is slightly offset outward from the central portion 37 of the fixation plate 35 (*i.e.,* is in a close parallel plane to the central portion 37). The top section 41 includes spaced apart fastener holes 42a, 42b, and a top central U-shaped cutaway section 43. The fixation plate 35 includes a bottom section 46 that is slightly offset outward from the central portion 37 of the fixation plate 35 (*i.e.,* is in a close parallel plane to the central portion 37). The bottom section 46 includes spaced apart fastener holes 47a, 47b, and a bottom central inverted U-shaped cutaway section 48. When used in spinal fusion, the walls 12, 16, 21, 25, 29, 33 of the cage 10 are positioned in the intervertebral space created by removal of the intervertebral disc between adjacent vertebrae. Fasteners are optionally inserted in fastener holes 42a, 42b for anterior attachment to a first upper vertebra, and fasteners are optionally inserted in fastener holes 47a, 47b for anterior attachment to an adjacent second lower vertebra. Top end surfaces 51, 52, 53, 54, 55, 56 of the walls 12, 16, 21, 25, 29, 33 would contact a lower surface of the first upper vertebra, and opposite bottom end surfaces of the walls 12, 16, 21, 25, 29, 33 would contact an upper surface of the second lower vertebra. The walls 12, 16, 21, 25, 29, 33 thereby provide mechanical load bearing support between the first upper vertebra and the second lower vertebra.

The vertical dimensions of the walls 12, 16, 21, 25, 29, 33 can be adjusted accordingly for various different intervertebral distances. Likewise, the horizontal length from the fixation plate 35 to the opposite outer end of each of the walls 12, 16, 21, 25, 29, 33 can be adjusted such that the ends of the walls 12, 16, 21, 25, 29, 33 do not extend outward beyond the perimeter of the first upper vertebra and the second lower vertebra. Similarly, the width of each of the walls 12, 16, 21, 25, 29, 33, and the width of each of the interior spaces 14, 18, 23, 27, and the width of each projection 13, 17, 22, 26, 31, 34 can be adjusted to control degradation characteristics. Optionally, the projections 13, 17, 22, 26, 31, 34 could attach adjacent walls. Also, the vertical and horizontal dimensions of the fixation plate 35 and the location of the fastener holes 42a, 42b, 47a, 47b can be varied to ensure proper location of the fastener holes 42a, 42b, 47a, 47b adjacent the first upper vertebra and the second lower vertebra for securing the cage 10 to the first upper vertebra and the second lower vertebra. By varying the vertical and horizontal dimensions of the walls 12, 16, 21 , 25, 29, 33 and the vertical and horizontal dimensions of the fixation plate 35, different size cages 10 can be provided for selection by a surgeon.

Because certain polymeric materials are degraded by physiological fluid, throughholes 38a, 38b, 38c, 38d are provided in the central portion 37 of the fixation plate 35 to allow fluid into the interior spaces 14, 18, 23, 27 of the cage 10 to degrade the walls 12, 16, 21 , 25, 29, 33 comprising the interior section of the cage 10. The throughholes serve to minimize any problems associated with tissue blockage of fluid. Optionally, flaps (not shown) can be provided on the top section 41 and the bottom section 46 of the fixation plate 35 to prevent backing out of the fasteners (e.g., fixation screws). In one example, the fixation screws are formed using the same biocompatible and biodegradable material with an osteoconductive mineral coating, and a bioactive agent associated with the biodegradable material and/or the coating.

In some embodiments, the cages provided herein comprise a porous biocompatible and biodegradable (if desired) material selected from polymeric materials, metallic materials, ceramic materials and mixtures thereof.

As used herein, a "biocompatible" material is one which stimulates at most only a mild, often transient, implantation response, as opposed to a severe or escalating response. As used herein, a "biodegradable" or "degradable" material is one which decomposes under normal in vivo physiological conditions into components which can be metabolized or excreted. As used herein, a bioactive agent is "associated" with the polymer and/or the coating if the bioactive agent is directly or indirectly, physically or chemically bound to the polymer and/or the coating. A bioactive agent may be physically bound to the polymer and/or the coating by entrapping, imbedding or otherwise containing a bioactive agent within the polymer and/or the coating network structure. A bioactive agent may be chemically bound to the polymer and/or the coating by way of a chemical interaction wherein a bioactive agent is covalently or noncovalently (e.g., by ionic interactions) bonded to the polymer and/or the coating. Thus, various techniques for associating a bioactive agent in or on the polymer and/or the coating are contemplated herein.

In certain embodiments, the spine fusion cages provided herein are formed from polycaprolactone, a biocompatible and biodegradable polymer. However, other polymers are known to be biocompatible, and can be used for the cages described herein. Nonlimiting examples of such polymers include polylactide, polyglycolide, poly(lactide-glycolide), poly(propylene fumarate), poly(caprolactone fumarate), polyethylene glycol, and poly(glycolide- co-caprolactone), polysaccharides (e.g. alginate), chitosan, polyphosphazene, polyacrylate, polyethylene oxide-polypropylene glycol block copolymer, fibrin, collagen, and fibronectin, polyvinylpyrrolidone, hyaluronic acid, polycarbonates, polyamides, polyanhydrides, polyamino acids, polyortho esters, polyacetals, polycyanoacrylates, polyurethanes, polyacrylates, ethylene- vinyl acetate polymers and other acyl substituted cellulose acetates and derivatives thereof, polystyrenes, polyvinyl chloride, polyvinyl fluoride, poly(vinylimidazole), chlorosulphonated polyolifins, polyethylene oxide, polyvinyl alcohol, teflon®, nylon, and analogs, mixtures, combinations and derivatives of any of the above.

In various examples, an osteoconductive mineral coating is formed on at least a portion of the cages provided herein. In some examples, the osteoconductive mineral coating comprises a plurality of discrete mineral islands; in other examples, the osteoconductive mineral coating comprises a substantially homogeneous mineral coating of the cages, or the non-fixation plate portion of the cages. In various embodiments, the mineral coatings may be any suitable coating material containing calcium and phosphate, such as hydroxyapatite, calcium-deficient carbonate-containing hydroxyapatite, tricalcium phosphate, amorphous calcium phosphate, octacalcium phosphate, dicalcium phosphate, calcium phosphate, and the like. The mineral coating may also include a plurality of layers having distinct dissolution profiles to control dissolution order, kinetics and delivery properties of any bioactive agents therein. Under physiological conditions, the solubility of calcium phosphate materials are as follows: amorphous calcium phosphate > dicalcium phosphate > octacalcium phosphate > tricalcium phosphate > hydroxyapatite (e.g., calcium-deficient carbonate-containing hydroxyapatite). Thus, a plurality of various calcium phosphate layers can provide a broad range of dissolution patterns. Incorporation of blank layers (i.e., calcium phosphate layers not containing any bioactive agent) can provide for delayed release.
Also, the incorporation of layers having different concentrations of bioactive agent can provide for varying release rates.

A bioactive agent can be associated with either or both of the uncoated biocompatible material forming the cages and/or the mineral coated portions of the cages provided herein.

A "bioactive agent" as used herein includes, without limitation, physiologically or pharmacologically active substances that act locally or systemically in the body. A bioactive agent is a substance used for the treatment, prevention, diagnosis, cure or mitigation of disease or illness, or a substance which affects the structure or function of the body or which becomes biologically active or more active after it has been placed in a predetermined physiological environment. Bioactive agents include, without limitation, enzymes, organic catalysts, nucleic acids including ribozymes and antisense RNA or DNA, organometallics, proteins (e.g., bone morphogenetic proteins including recombinant human bone morphogenetic proteins), demineralized bone matrix, bone marrow aspirate, glycoproteins, peptides, polyamino acids, antibodies, nucleic acids, steroidal molecules, antibiotics, antimycotics, cytokines, fibrin, collagen, fibronectin, vitronectin, hyaluronic acid, growth factors (e.g., transforming growth factors and fibroblast growth factor), carbohydrates, statins, oleophobics, lipids, extracellular matrix and/or its individual components, pharmaceuticals, and therapeutics. However, the calcium phosphate coatings described above are not defined as bioactive agents herein.

In some embodiments, the bioactive agent is a growth factor such as growth hormone (GH); parathyroid hormone (PTH, including PTH1-34); bone morphogenetic proteins (BMPs) such as BMP2A, BMP2B, BMP3, BMP4, BMP5, BMP6, BMP7 and BMP8; transforming growth factor-α (TGF-α), TGF-β1 and TGF-β2; fibroblast growth factor (FGF), granulocyte/macrophage colony stimulating factor (GMCSF), epidermal growth factor (EGF), platelet derived growth factor (PDGF), growth and development factor-5 (GDF-5), an insulin-like growth factor (IGF), leukemia inhibitory factor (LIF), vascular endothelial growth factor (VEGF), basic fibroblast growth factor (bFGF), platelet derived growth factor (PDGF), angiogenin, angiopoietin-1, del-1, follistatin, granulocyte colony-stimulating factor (G-CSF), hepatocyte growth factor/scatter factor (HGF/SF), interleukin-8 (IL-8), leptin, midkine, placental growth factor, platelet-derived endothelial cell growth factor (PD-ECGF), platelet-derived growth factor-BB (PDGF-BB), pleiotrophin (PTN), progranulin, proliferin, tumor necrosis factor-α (TNF-α), vascular endothelial growth factor (VEGF), a matrix metalloproteinase (MMP), angiopoietin 1 (ang1), ang2, or delta-like ligand 4 (DLL4).

In some specific embodiments, the bioactive agent is a BMP such as BMP2, BMP4, BMP7, or BMP14, an IGF, an FGF, a PDGF, GDF-5, a TGF, a VEGF or platelet rich plasma (PRP).

Different release rates of the bioactive agent would be possible from uncoated and coated areas of the cages provided herein. See also PCT Patent Application PCT/US09/58419, entitled MINERAL-COATED MICROSPHERES, filed September 25, 2009 for a discussion of other ways to affect the release rate of a bioactive agent from a calcium phosphate coating, including building up layers of the coating with different dissolution patterns, or binding a component to the coating that provides a functional group to which the bioactive agent can be covalently bound.

While various bioactive agents listed above are suitable for use with the cage 10, in one embodiment, the bioactive agent is selected from bone morphogenetic proteins (BMPs), demineralized bone matrix, bone marrow aspirate, and mixtures thereof. BMPs have been shown to be excellent at growing bone and powdered recombinant human BMP2 is available in certain commercial products. Demineralized bone matrix includes osteoinductive proteins (e.g., BMPs), and can be used in a particle or fiber form. Bone marrow aspirate contains osteoprogenitor cells, and the patient's bone marrow can be readily harvested with a needle.

The bioactive agent is present in an amount that induces ossification between the adjacent bones or adjacent bone surfaces. The amount of bioactive agent included on uncoated and/or coated areas of the cage 10 will depend on a variety of factors including the nature of the bioactive agent, the osteoinductive potential of the bioactive agent, and the nature of the carrier material (e.g., the biocompatible material forming the cage 10 or the mineral coating on the cage 10). Investigations have shown that a 1-100 ng/ml concentration of BMP can induce osteogenesis; and in one example, the BMP in the present invention can be released from the cage 10 in a time frame that varies from 10-50 days. Therefore, without intending to limit the invention in any way, in the case of bone morphogenetic proteins, it is contemplated that in one example a concentration of about 10-5000 ng of bone morphogenetic protein per cm³ of material would be suitable for inducing ossification between the adjacent bones or adjacent bone surfaces.

Various regions of the cages can include the coatings and/or bioactive agent. In some examples, e.g. top and bottom end regions of the walls 12, 16, 21 , 25, 29, 33 that are positioned near the opposed vertebrae are coated with a continuous layer or islands of the coating and associated bioactive agent so that bone growth is induced, while interior sections of the cage might not include coatings and associated bioactive agent in order to promote growth of fibrous tissue. As an exemplary illustration, top end surfaces 51, 52, 53, 54, 55, 56 in FIG. 1B could include a continuous mineral coating and associated bioactive agent so that bone fixation to the adjacent vertebra is induced, while regions near the projections 13, 17 22, 26, 31, 34 may not include the coating and associated bioactive agent so fibrous growth is promoted in that region.

In some examples, the bioactive agent (e.g., bone morphogenetic protein) is associated with uncoated biocompatible material forming the cage 10 and/or the mineral coated portions of the cage 10 prior to inserting the walls 12, 16, 21, 25, 29, 33 of the cage 10 in the intervertebral disc space. For example, a bone morphogenetic protein may be chemically bonded (e.g., ionically or covalently bonded) to a calcium phosphate coating at a manufacturing site, or alternatively a bone morphogenetic protein may be chemically bonded to the calcium phosphate coating by a surgeon before and/or after implantation. The surgeon can reconstitute powdered bone morphogenetic protein with sterile water and apply the reconstituted powdered bone morphogenetic protein to the cage 10. It is contemplated that the calcium phosphate layer can be selected to best accept BMP-2 applied by a surgeon.

Any of the cages described herein can also be seeded with a mammalian cell, either before the cage is implanted, or during implantation. The cell can be derived from the intended recipient of the cage, or from another donor. Additionally, the cell can be a primary cell, i.e., taken from the donor without culture, or the cell could be cultured any length of time prior to seeding. Further, the cage can be seeded with cells then incubated under appropriate conditions to allow colonization of the cage to any degree prior to implant.

In some examples, the cell is a terminally differentiated cell, e.g., an osteoblast. In other examples, the cell is less differentiated, for example a stem cell such as an embryonic stem cell or an adult stem cell, e.g., a mesenchymal stem cell. In various examples, the stem cell is derived from a cell isolated in the undifferentiated state. In alternative examples, the stem cell is induced (known as induced pluripotent stem cells or iPS cells) from a differentiated cell, by any means known in the art (e.g., by transfection with a transgene or by treatment with a cytokine).

In some examples, various optional features of the cage 10 are beneficial. Because placement of the cage 10 may be performed using a medical imaging device and techniques (e.g., fluoroscopic observation), the cage 10 may further include at least one marking including a tracer that provides enhanced visibility via the medical imaging device. Non-limiting examples of radiopaque materials for enhanced visibility during fluoroscopy include barium sulfate, tungsten, tantalum, zirconium, platinum, gold, silver, stainless steel, titanium, alloys thereof, and mixtures thereof. Radiopaque markings can be used as an alignment aid in verifying the proper positioning of the cage 10. Also, the cage 10 may include a region of no material or radiolucent material such that the region forms an imaging window for enhanced visibility through the imaging window via a medical imaging device. The image-based design methods as in U.S. 2003/0069718 are beneficial as the imaging window can be arranged in the cage without compromising the strength of the cage.

Based on the above discussion, the cage illustrated in FIG. 1 can, in certain embodiments, be described as comprising a designed porous microstructure comprising a biocompatible material; a plurality of substantially parallel spaced apart walls, the walls interconnected by transverse projections; and a fixation plate comprising a central portion, where the walls are coupled to the central portion of the fixation plate, where the walls are substantially perpendicular to the central portion of the fixation plate and the walls have a substantially rectangular transverse vertical cross section; and the cage is designed to fit the parallel spaced apart walls into an intervertebral space created by removal of an intervertebral disc between adjacent cervical vertebrae. The cage further comprises a top section of the fixation plate and a bottom section of the fixation plate, where the top section of the fixation plate is above and in the same plane or a close parallel plane as the central portion of the fixation plate, and the bottom section of the fixation plate is below and in the same plane or a close parallel plane as the central portion of the fixation plate, and where the top section of the fixation plate and the bottom section of the fixation plate each further comprise fastener holes suitable for accommodating fasteners to affix the cage to the adjacent bones. The biocompatible material of the cage is polycaprolactone; and the cage further comprises a coating of calcium-deficient carbonate-containing hydroxyapatite that substantially homogeneously covers the plurality of parallel spaced apart walls and transverse projection.

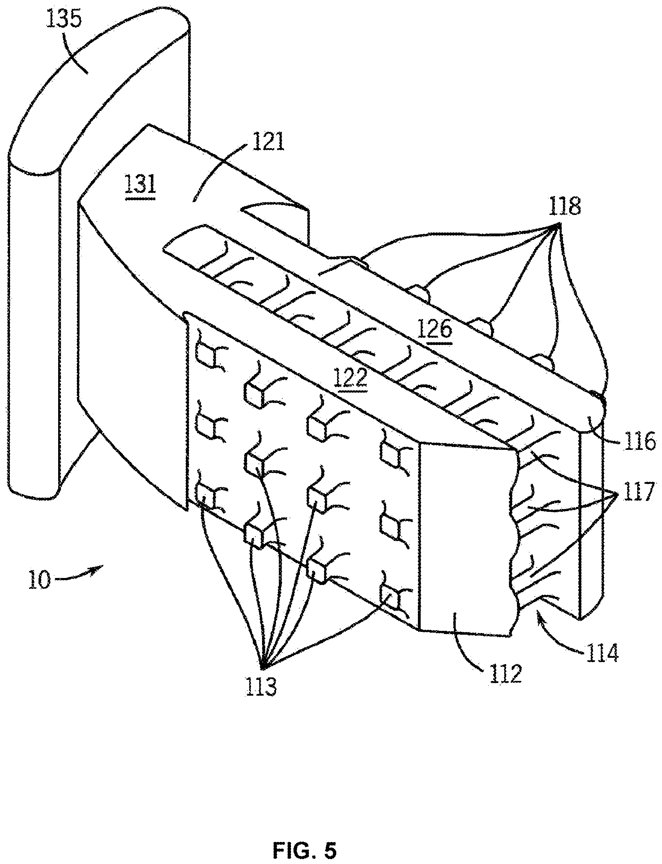

For lumbar fusion, a transforaminal lumbar interbody fusion (TLIF) cage is provided with an integrated lateral plate for fixation. Thus, only a one step procedure is needed to implant a cage with associated fixation. One example of this design is shown in FIG. 5.

Looking now at FIG. 5, there is shown an image-based porous microstructure design of a transforaminal lumbar interbody fusion cage 110. The cage 110 has a first vertical wall 112 having a substantially rectangular transverse vertical cross section. The first wall 112 has projections 113 extending substantially perpendicularly from a vertical side surface of the first wall 112. A first space 114 is created between the first wall 112 and a second vertical wall 116 having a substantially rectangular transverse vertical cross section. The second wall 116 has projections 117 extending substantially perpendicularly from a vertical side surface of the second wall 116. The second wall 116 also has projections 118 extending substantially perpendicularly from an opposite vertical side surface of the second wall 116. The first wall 112 and the second wall 116 are connected to a base section 121.

Still referring to FIG. 5, the cage 110 has a slightly arcuate fixation plate 135. The walls 112, 116 and the base section 121 are substantially perpendicular with the fixation plate 135. The fixation plate 135 can include spaced apart fastener holes (not shown) in a top section of the fixation plate 135 and spaced apart fastener holes (not shown) in a bottom section of the fixation plate 135 as in the cage 10 of FIGS. 1A and 1B. The fixation plate 135 can also include throughholes (as in the cage 10 of FIGS. 1A and 1B) in the central portion of the fixation plate 135 to allow fluid into the interior space 114 of the cage 110 to degrade the walls 112, 116 comprising the interior section of the cage 110. When used in spinal fusion, the walls 112, 116, of the cage 110 are positioned in the intervertebral space created by removal of the intervertebral disc between adjacent vertebrae. Fasteners are optionally used for lateral attachment of the fixation plate 135 to a first upper vertebra and adjacent second lower vertebra. Top end surfaces 122, 126, 131 of the walls 112, 116 and the base section 121 would contact a lower surface of the first upper vertebra, and opposite bottom end surfaces of the walls 112, 116 and the base section 121 would contact an upper surface of the second lower vertebra. The walls 112, 116 and the base section 121 thereby provide mechanical load bearing support between the first upper vertebra and the second lower vertebra. As in the cage 10, the vertical and horizontal dimensions of the walls 112, 116, and the vertical and horizontal dimensions of the fixation plate 135 can be varied so that different size cages 110 can be provided for selection by a surgeon.

In some embodiments, the cage 110 comprises a porous biocompatible, biodegradable (if desired) material selected from polymeric materials, metallic materials, ceramic materials and mixtures thereof. In various embodiments, the spine fusion cage 110 is formed from polycaprolactone, a biocompatible and biodegradable polymer. However, other polymers such as polylactide, polyglycolide, poly(lactide-glycolide), poly(propylene fumarate), poly(caprolactone fumarate) and poly(glycolide-co-caprolactone) may also be advantageous for forming the cage 110.

In some embodiments, an osteoconductive mineral coating is formed on at least a portion of the cage 110. The osteoconductive mineral coating can comprise a plurality of discrete mineral islands, or the mineral coating can be formed on the entire surface of the cage 110. In one exemplary form, the osteoconductive mineral coating comprises a substantially homogeneous mineral coating. In other embodiments, the mineral coatings may be any suitable coating material containing calcium and phosphate, such as hydroxyapatite, calcium- deficient carbonate-containing hydroxyapatite, tricalcium phosphate, amorphous calcium phosphate, octacalcium phosphate, dicalcium phosphate, calcium phosphate, and the like. The mineral coating may also include a plurality of layers having distinct dissolution profiles to control dissolution order, kinetics and bioactive delivery properties as in the cage 10.

A bioactive agent can be associated with uncoated biocompatible material forming the cage 1 10 and/or the mineral coated portions of the cage 1 10. Different release rates of the bioactive agent would be possible from uncoated and coated areas of the cage 1 10. The bioactive agent is present in amount that induces ossification between the adjacent bones. While various bioactive agents listed above are suitable for use with the cage 1 10, in some embodiments the bioactive agent is selected from bone morphogenetic proteins, demineralized bone matrix, bone marrow aspirate, and mixtures thereof.

In various examples, features of the cage 1 10 include at least one marking including a tracer that provides enhanced visibility via a medical imaging device can be located on the cage 1 10. Specifically, at least one radiopaque marking that provides enhanced visibility via a fluoroscope can be located on the cage 1 10. The cage 1 10 can include a region of no material or radiolucent material such that the region forms an imaging window for enhanced visibility through the imaging window via a medical imaging device. Also, the cage can include at least one marking for alignment during implantation.

The methods of U.S. Patent Application Publication No. 2006/0276925 also provide a design methodology for creating biomaterial scaffolds with internal porous architectures that meet the need for mechanical stiffness and strength and the need for connected porosity for cell migration and tissue regeneration. The methods of U.S. 2006/0276925 can be used to generate a lumbar spine interbody fusion cage with a designed periodic microstructure that attains desired stability (displacements <0.9 mm), while maintaining compliance to avoid stress shielding and a large porosity for biofactor delivery.

Based on the above discussion, an alternative cage illustrated in FIG. 5 can be described as comprising a designed porous microstructure comprising a biocompatible material; a plurality of substantially parallel spaced apart
walls, the walls interconnected by transverse projections (117);
and a fixation plate
comprising a central portion, wherein the walls are coupled to the central portion of the fixation plate, wherein the walls are coupled to the central portion through a base section that is substantially perpendicular to the central portion, and the central portion extends above and below the base section and walls and is slightly arcuate. The cage is designed to fit the parallel spaced apart walls into an intervertebral space created by removal of an intervertebral disc between adjacent lumbar vertebrae; the biocompatible material is polycaprolactone; and the cage further comprises a coating of calcium-deficient carbonate-containing hydroxyapatite that substantially homogeneously covers the plurality of parallel spaced apart walls and transverse projections.

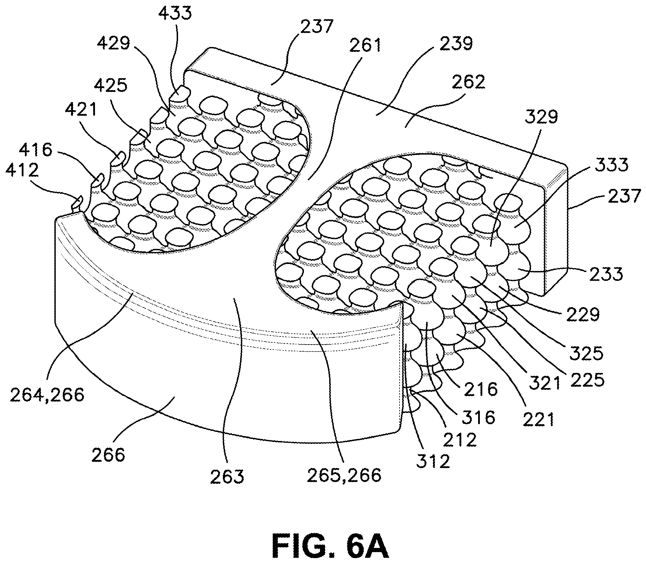

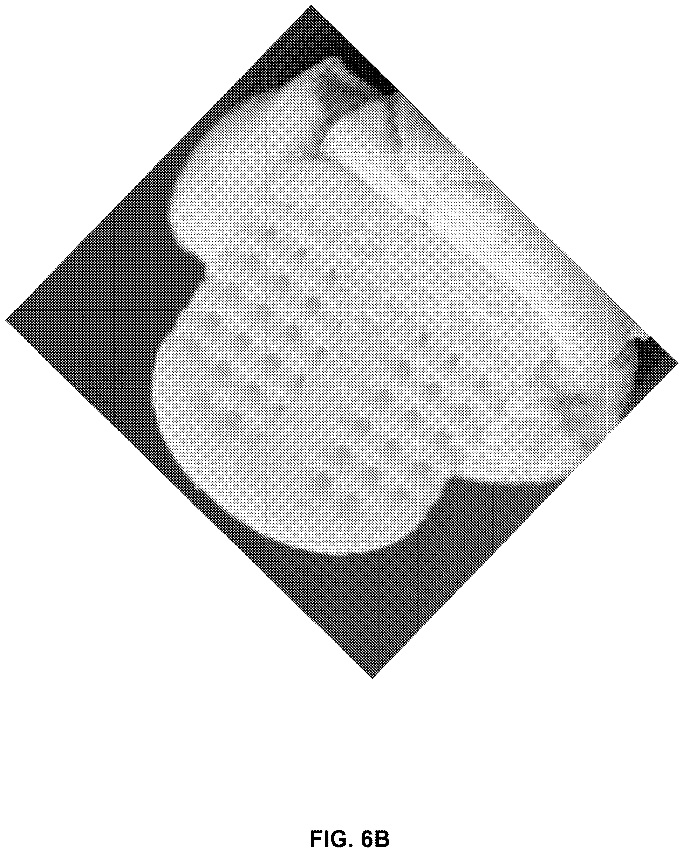

FIG. 6 shows a cage design 210 in accordance with embodiments of the invention, which can be made using any of the same methods and materials as with any of the cages described above. As with the cages described above, this design comprises a designed porous microstructure comprising a biocompatible material; a central portion 237 of a fixation plate; and a plurality of parallel spaced apart walls 212, 216, 221 , 225, 229, 233, 312, 316, 321 , 325, 329, 333, 412, 416, 421 , 425, 429, 433 (collectively 212, etc.). In various examples, the walls 212, etc., are interconnected by transverse projections (shown in FIG. 6B). The walls can be short and stacked on top of each other (e.g., wall 312 is on top of wall 212). In such examples, the walls will have a substantially circular, oval, ovoid, or polygonal transverse vertical cross section. Alternatively, the walls can extend the entire thickness of the cage 210, as in the cages 10,1 10 described above and illustrated in FIGS. 1 , 2 and 5. In those alternative examples, the walls will have a substantially rectangular transverse vertical cross section.

In the cage design illustrated in FIG. 6, the walls 212, etc., are substantially parallel to the central portion 237 and the walls 212, etc., are joined to the central portion 237 of the fixation plate through at least one support 261 having a proximal end 262 and a distal end 263, the support 261 joined at the proximal end 262 to the central portion 237 and extending perpendicularly therefrom. The support 261 can extend from any point in the central portion. In the illustrated example, the central portion 237 comprises a midpoint 239 and the cage comprises one support 261 extending from the midpoint 239 of the central
portion 237. In various embodiments, the cage further comprises at least two flanges 264, 265 extending from the distal end of the support 261. In the illustrated embodiments, each flange 264, 265 extends in a direction such that the walls 212, etc., are positioned between the flanges 264, 265 and the central portion 237, where the flanges 264, 265 are substantially coextensive with the walls 212, etc., and the central portion 237. In these examples, the flanges 264, 265 are substantially perpendicular to the support 261 , and coupled to form an inwardly directed arcuate member 266. These alternative cages are particularly useful when designed for the parallel spaced apart walls to fit into an intervertebral space created by removal of an intervertebral disc between adjacent cervical vertebrae, however, the design could be used for other fusions, e.g., other vertebrae, or to fill the gap of a bone defect.

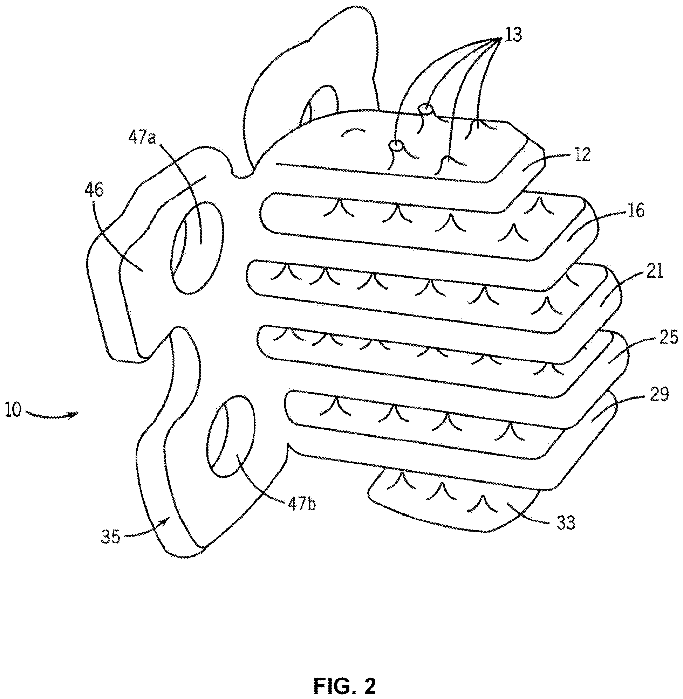

In various examples, the cage further comprises fixation plate components to affix the cage to the adjacent bones, for example a top section 41 of the fixation plate and a bottom section 46 of the fixation plate, as shown in FIGS. 1 and 2, where the top section 41 of the fixation plate is above and in the same plane or a close parallel plane as the central portion 37, 237 of the fixation plate, and the bottom section 46 of the fixation plate is below and in the same plane or a close parallel plane as the central portion 37 of the fixation plate. In some examples, the top section 41 of the fixation plate and the bottom section 46 of the fixation plate each further comprise fastener holes 42, 47 suitable for accommodating fasteners to affix the cage to the adjacent bones. In some embodiments of these cages, the biocompatible material is polycaprolactone. In other examples, the cage further comprises a coating of calcium-deficient carbonate-containing hydroxyapatite that substantially homogeneously covers the plurality of parallel spaced apart walls and transverse projections. In additional embodiments, the cage further comprises a bioactive agent, where the bioactive agent is present in an amount that induces ossification between the adjacent bones. As discussed above, in some embodiments, the bioactive agent is a bone morphogenetic protein (BMP), demineralized bone matrix, a bone marrow aspirate, a transforming growth factor, a fibroblast growth factor, an insulin-like growth factor, a platelet derived growth factor, a vascular endothelial growth factor, a growth and development factor-5, platelet rich plasma, or a mixture thereof. In particular embodiments, the bioactive agent is BMP2 or BMP7.

### Cage Fabrication

Once the intervertebral scaffolding image-design dataset is created, it can be automatically converted into a surface representation in .stl file format (stereolithography triangular facet data). This makes it possible to fabricate the intervertebral scaffolding from any type of Solid Free-Form Fabrication (SFF) system using either direct or indirect methods. Direct SFF methods include, but are not limited to: (1) Selective Laser Sintering (SLS); (2) Stereolithography (SLA); (3) Fused Deposition Modeling (FDM); and (4) Selective Laser Melting (SLM). In the present invention, both of the conventional design of the tapered cage and the new design by degradation topology optimization can be exported to an EOS Formega P 100 machine (3D Systems, Valencia, California, USA) in .stl file format, and can be used to construct scaffolds by SLS processing of ε-polycaprolactone powder. This particular form of polycaprolactone has a melting point of 60 °C, a molecular weight in the range of 35,000 to 100,000 Daltons, and particle size distribution in the 25-100 pm range. However, nanoscale particle sizes are also suitable in place of the microscale particle sizes. SLS processing of the polycaprolactone powder can be conducted by preheating the powder to 49.5 °C and scanning the laser (450 µηι focused beam diameter) at 4.5 Watts power and 1 .257 m/s (49.5 inches/s) scan speed. Cages can be built layer-by-layer using a powder layer thickness of 100 pm. After SLS processing is completed, the cages can be allowed to cool inside the machine process chamber for approximately 1 hour and can then be removed from the part bed. Excess powder surrounding the cages will be brushed off and the cages will be finally cleaned by blowing compressed air and physically removing unsintered powder from the cage interstices by insertion of a 1 millimeter diameter wire. FIG. 2 is an illustration of a fabricated polycaprolactone cervical cage prototype that can be built using the SLS process. (The reference numerals of FIGS. 1A and 1B have been applied to FIG. 2.) In an alternative method, calcium-phosphate-based particles or fibers are included with the polycaprolactone powder before sintering such that the calcium-phosphate-based particles or fibers are dispersed in the final formed cage. The particle sizes for the calcium-phosphate-based particles can be nanoscale or microscale.

### Develop and Characterize Calcium Phosphate-Based Mineral Coatings on Polycaprolactone cages

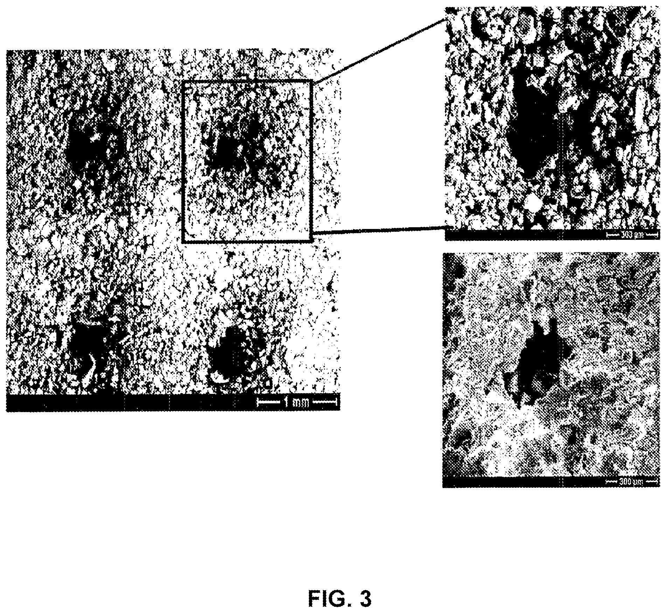

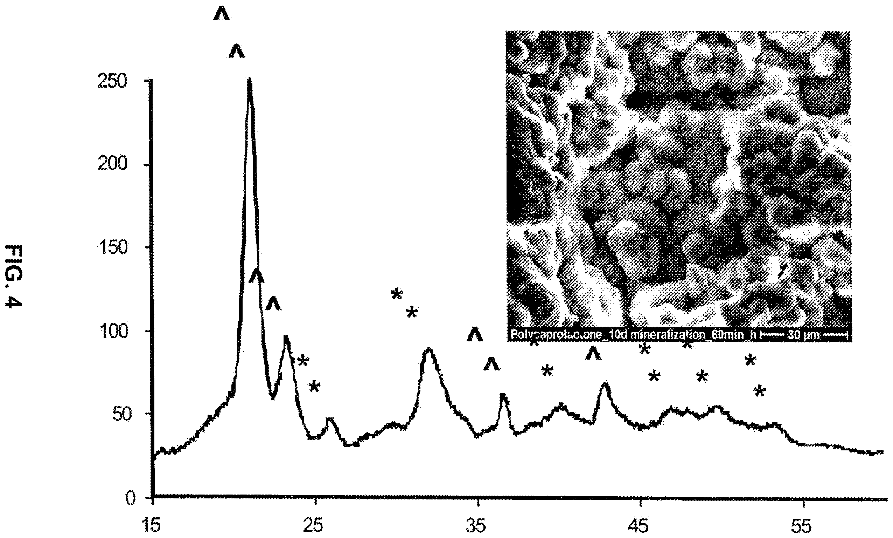

To induce formation of a calcium phosphate-based mineral layer, polycaprolactone samples are, in some examples, incubated in modified simulated body fluid (mSBF) solutions for mineral nucleation and growth. The mSBF solution contains the ionic constituents of blood plasma, with double the concentrations of calcium and phosphate ions, held at physiologic temperature and pH 6.8. The growth of calcium phosphate-based minerals, specifically bone-like minerals, on bioresorbable polymer matrices using mSBF incubation has been demonstrated (see, Lin et al. "A novel method for internal architecture design to match bone elastic properties with desired porosity", Journal of Biomechanics 37:623-36, 2004; Murphy et al., "Bioinspired growth of crystalline carbonate apatite on biodegradable polymer substrata", J Am Chem Soc 124:1910-7, 2002; and Murphy et al., "Effects of a bone-like mineral film on phenotype of adult human mesenchymal stem cells in vitro", Biomaterials 26:303-10, 2005). Looking at FIGS. 3 and 4, the results of coating polycaprolactone with calcium phosphate are shown. FIG. 3 shows scanning electron microscope (SEM) micrographs showing large scale pore structure of polycaprolactone scaffolds without calcium phosphate coating (left panel, top right image), and with calcium phosphate coating (bottom right image).
FIG. 4 shows an X-ray diffraction spectrum showing hydroxyapatite (*) grown on a polycaprolactone (^) scaffold. The inset is a high magnification SEM image of a calcium phosphate coating grown on a polycaprolactone scaffold. Therefore, this serves as a reliable method for growth of mineral coatings.

Mineral formation in mSBF can be tracked by analyzing changes in solution calcium concentration using a calcium sensitive electrode (Denver Instrument, Denver, CO). After their growth, the mineral matrices can be dissolved and analyzed for calcium and phosphate ion content to quantify mineral formation, and the mineral crystals can be analyzed morphologically and compositionally using a scanning electron microscope (SEM), *e.g.,* with a Noran SiLi detector for elemental analysis. The chemical composition can be further analyzed using Fourier transform infrared spectroscopy to identify phosphate bond vibrations (570 cm⁻¹, 962 cm⁻¹, and 1050 cm⁻¹). Dissolution of mineral layers can also be characterized by measuring release of calcium and phosphate ions during incubation in tris-buffered saline at 37 °C at pH 7.4. Calcium and phosphate concentrations can be measured using previously described colorimetric assays (see Murphy et al., "Bioinspired growth of crystalline carbonate apatite on biodegradable polymer substrata", J Am Chem Soc 124:1910-7, 2002). Each of the characterization methods described in this section is routine in analysis of inorganic materials, and is consistent with FDA's good guidance practices for design and testing of calcium phosphate coatings (see Devices FDoGaR. Calcium phosphate coating draft guidance for preparation of FDA submissions for orthopedic and dental endosseous implants. 1997).

It is thus possible to confer both osteoconductivity and osteoinductivity to orthopedic implant materials using calcium phosphate coatings. Based on the well-defined osteoconductivity and potential osteoinductivity of calcium-phosphate-based mineral coatings, calcium phosphate mineral growth can be advantageously utilized to coat polycaprolactone spine fusion cages.

### Incorporate Bioactive Agent Within and Upon Growing Mineral Coatings and Evaluate Incorporation and Release

Prior to *in vivo* experiments to test the efficacy of any fusion cages, *in vitro* studies can be performed to validate the approach. The focus of the *in vitro* work can be, *e.g.,* directed toward understanding the interaction between a bioactive agent (*e.g.,* a bone morphogenetic protein such as BMP2 or BMP7) and calcium phosphate mineral coatings, measuring the release of the bioactive agent from the coatings, and confirming biological activity of released bioactive agent. The following paragraphs delineate specific *in vitro* experiments.

Binding of bioactive agent to mineralized PCL scaffolds followed by release: To characterize binding of a bioactive agent, e.g., BMP2, to calcium phosphate mineral coatings, ¹²⁵I-labeled BMP2 (e.g., from ICN Biomedicals) can be used. Radiolabeling represents a highly sensitive and convenient method for characterizing protein binding and release (see, e.g., Murphy et al., "Bone regeneration via a mineral substrate and induced angiogenesis", J Dent Res 2004;83:204-10; and Murphy et al., "Growth of continuous bonelike mineral within porous poly(lactide-co- glycolide) scaffolds in vitro",. J Biomed Mater Res 50:50-8, 2000). Mineral coatings can be grown on polycaprolactone cages, followed by an incubation (e.g., for 4 hours) in solutions containing 1- 100 nM ¹²⁵I-labeled BMP2. The calcium-phosphate coated scaffolds are expected to bind BMP2 with 50-100% efficiency in the soluble BMP2 concentration range explored (see, Gittens et al., "Imparting bone mineral affinity to osteogenic proteins through heparin-bisphosphonate conjugates", J Control Release 98:255-68, 2004). The scaffolds can then be removed from solution, rinsed with serum free DMEM, and analyzed for radioactivity using a scintillation counter. To characterize subsequent release of a bound bioactive agent such as BMP2, the samples can be incubated in DMEM with 10% FBS for 14 days. In some examples, media is refreshed, e.g., every 24 hr., and radioactivity in solution can be measured. It is expected that the release will primarily take place over the initial 7 days in solution with near zero order release kinetics (Gittens et al., "Imparting bone mineral affinity to osteogenic proteins through heparin-bisphosphonate conjugates", J Control Release 98:255-68, 2004). These experiments can also demonstrate release of a broad range of total BMP2 from scaffolds, as the total amount of protein released will be dictated by the amount of BMP2 present in the binding solution (1-100 nM). It is contemplated that many bioactive agents, in particular protein bioactive agents such as BMP2, form ionic bonds to hydroxyapatite in solution. It is also contemplated that certain amino acid mineral binding fragments could be incorporated into the BMP such that the mineral binding fragments form ionic bonds to hydroxyapatite in solution. Ionic binding advantageously provides suitable controlled delivery of bone morphogenetic protein through dissolution of the calcium phosphate layer or degradation of the polycaprolactone. In contrast, sponge based BMP delivery systems rely on absorption of the BMP into the sponge which makes controlled delivery difficult to attain.

Examining Biological Activity of Engineered Growth Factors: In order to confirm the biological activity of BMP after binding to, and release from, mineral layers, an assay should be well-defined and biologically relevant. Promotion of osteogenic differentiation of multipotent cell types is a key function of several BMPs (see Nakamura et al., "p38 mitogen-activated protein kinase functionally contributes to chondrogenesis induced by growth/differentiation factor-5 in ATDC5 cells", Exp Cell Res 250:351-63, 1999; Saito et al., "Activation of osteoprogenitor cells by a novel synthetic peptide derived from the bone morphogenetic protein-2 knuckle epitope", Biochim Biophys Acta 1651:60-7, 2003; and Saito et al., "Prolonged ectopic calcification induced by BMP-2-derived synthetic peptide", J Biomed Mater Res A 70A:115-21, 2004), and osteogenic induction of the mouse embryonic fibroblast cell line C3H10T1/2 by BMP2 is well-characterized. Therefore, a cell-based biological activity assay, e.g., using C3H10T1/2 when the bioactive agent is BMP2, can be used to characterize soluble bioactive agent such BMP2 released from mineralized polycaprolactone scaffolds. For example, cells can be exposed to 0.1-100 ng/ml BMP-2 released from mineral layers and measure alkaline phosphatase upregulation, a hallmark of osteogenic induction by BMP2, using a standard colorimetric assay. These results can be compared to a standard curve that relates soluble BMP2 concentrations (not released from scaffolds) to alkaline phosphatase upregulation, which will give the effective activity of BMP2 released from scaffolds. The activity of the released BMP-2 is not expected to be substantially effected by mineral binding and release, as BMPs are known to bind strongly to calcium phosphate minerals under normal conditions *in vivo* (see, Gorski et al., "Is all bone the same? Distinctive distributions and properties of non-collagenous matrix proteins in lamellar vs. woven bone imply the existence of different underlying osteogenic mechanisms", Crit Rev Oral Biol Med 9:201-23, 1998; Gorski et al., "Bone acidic glycoprotein-75 is a major synthetic product of osteoblastic cells and localized as 75- and/or 50-kDa forms in mineralized phases of bone and growth plate and in serum", J Biol Chem 265:14956-63, 1990).

Therefore, it can be seen that the invention provides a cage for facilitating the fusion of adjacent bones such as vertebrae, or adjacent bone surfaces, such as in an open fracture. In one form, the cage includes a plurality of spaced apart walls comprising porous polycaprolactone; an osteoconductive mineral coating (*e.g.,* a calcium phosphate compound) on at least a portion of the walls; and a bioactive agent (*e.g.,* a bone morphogenetic protein) associated with the polycaprolactone and/or the coating. In these embodiments, the bioactive agent is present in an amount that induces ossification between the adjacent bones or adjacent bone surfaces. The cage can also include a fixation plate connected to at least one of the walls, wherein the fixation plate also comprises polycaprolactone.

Although the invention has been described in considerable detail with reference to certain embodiments, one skilled in the art will appreciate that the present invention can be practiced by other than the described embodiments, which have been presented for purposes of illustration and not of limitation. For instance, while the cage of the invention is advantageous in the fixation, connecting, reconstruction and/or regeneration of vertebrae, the cages of the invention would be suitable for the fusion of any adjacent bones or adjacent bone surfaces. For example, the cages of the invention could be used in the treatment of acute, open fractures in a bone (e.g., tibia), or in oral and maxillofacial bone grafting procedures. Therefore, the scope of the appended claims should not be limited to the description of the embodiments contained herein.

Preferred embodiments are described in the following example. Other embodiments within the scope of the claims herein will be apparent to one skilled in the art from consideration of the specification or practice of the invention as disclosed herein. It is intended that the specification, together with the examples, be considered exemplary only.

Example - In vivo fusion using polycaprolactone cages in a large animal model

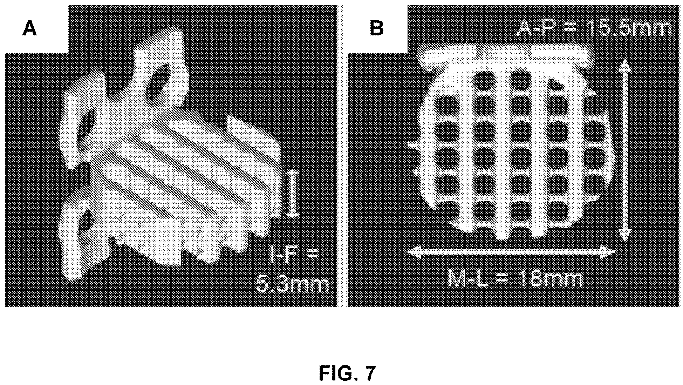

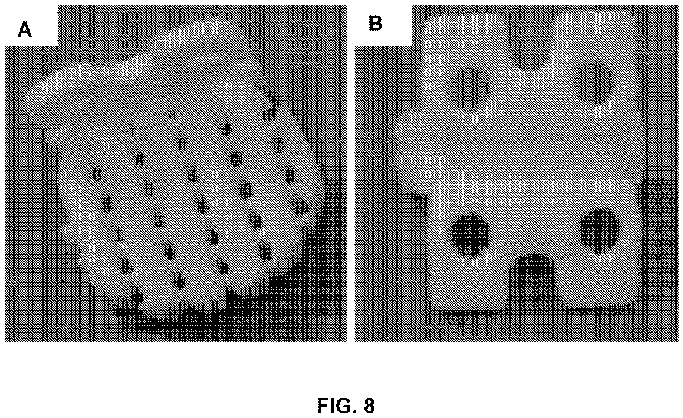

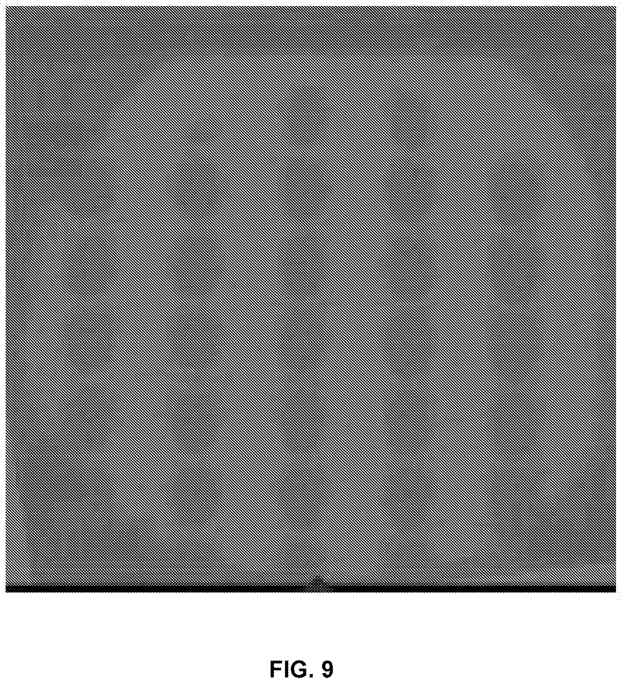

Cages were designed and constructed using the methods described above. FIG.
7 shows the design model of the cages, and FIG. 8 shows the constructed cage. The interbody portion was 5.3 mm thick, having "fingers" designed to withstand surgical implantation loading in addition to in vivo compression forces and bending moments (FIG. 7). The plate was designed with four screw holes for surgical attachment (FIG. 7). These fusion device sizes were similar to human cage designs, a rationale for using the pig as an animal model. The manufacturing process can readily build 30 cages per day on one machine. Manufacturing quality was assessed both non-destructively using micro-computed tomography (micro-CT) scanning and mechanical compression testing. Micro-CT analysis (FIG. 9) demonstrated fidelity of manufactured device to the design with a defect volume of less than 0.6%.

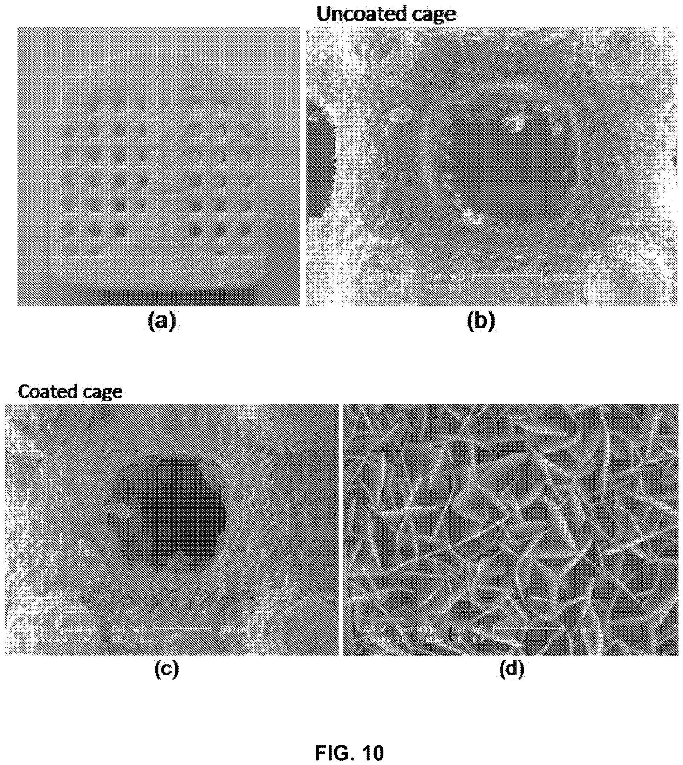

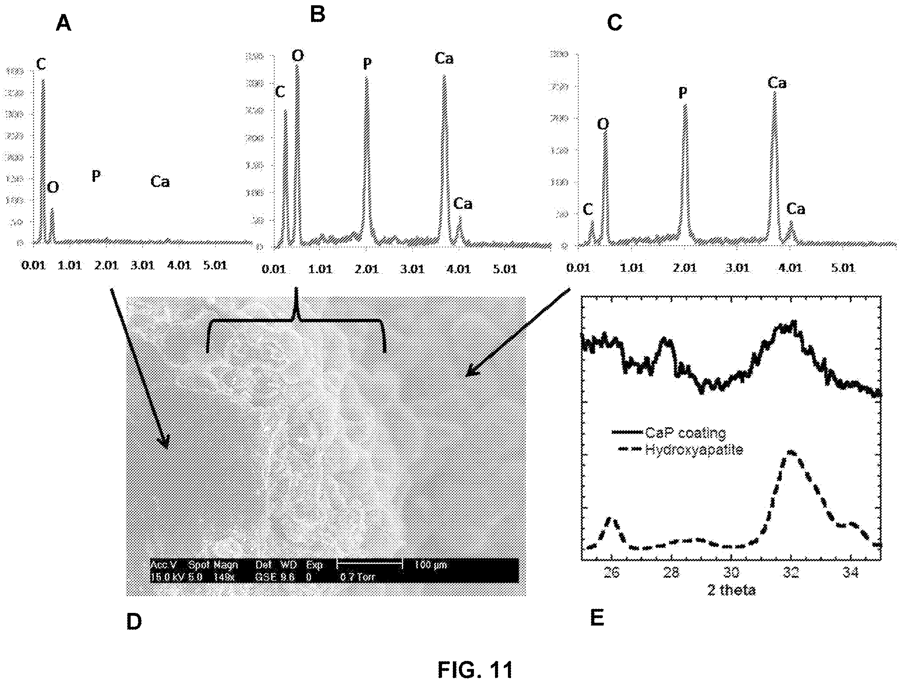

Some of the cages were coated with a 50-100 micron thick calcium phosphate coating using the method described in, e.g., Murphy et al., "Bioinspired growth of crystalline carbonate apatite on biodegradable polymer substrata", J Am Chem Soc 124:1910-7, 2002, and PCT Patent Application PCT/US09/58419, entitled MINERAL-COATED MICROSPHERES, filed September 25, 2009 (FIG. 10). The coating contained a Ca to P ratio of 1.3, similar to the Ca/P ratio of tricalcium phosphate and hydroxyapatite (FIG. 11).

Compression testing of the interbody portion (N=4) gave a yield load of 1620 ± 20 Newtons (N) and an effective modulus of 95 ± 3.6 MPa. These results demonstrate a very consistent and reproducible manufacturing process that produces devices with standard deviations of yield load of 1.2% of the mean and effective modulus of 3.8% of the mean. The compressive yield load of this design fusion mesh is greater than 10 times typical cervical spine compression loads of 150 N. The elastic moduli were very similar to vertebral trabecular bone, thus reducing the chance of subsidence.

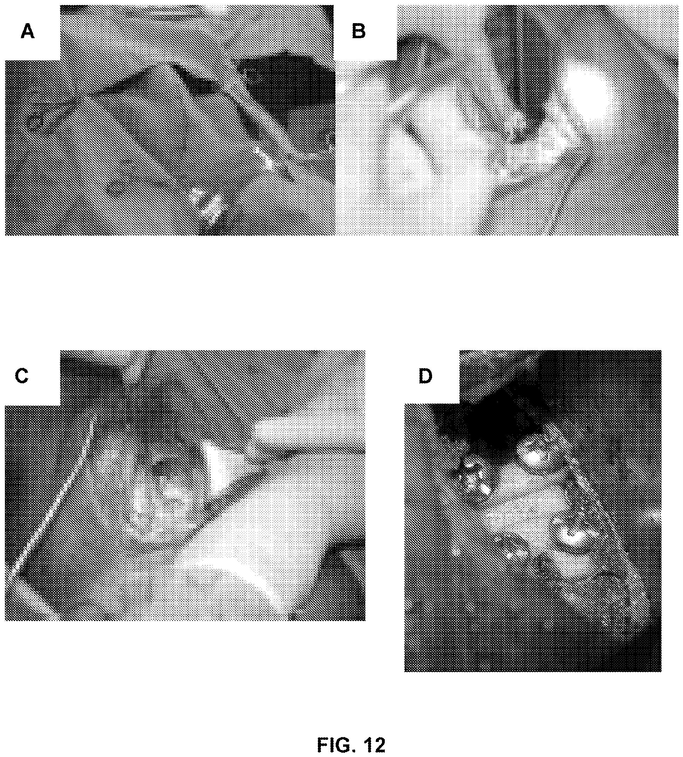

Cages were implanted into 11 6-9 month old Yucatan minipigs at the C5-C6 level (FIG. 12) according to the protocol outlined in Table 1, using a standard anterior approach. The animals were sacrificed at 6 months, 12 months or 18 months after implant (Table 1). Group 1 cages had the calcium phosphate coating; Group 2 cages did not have a coating but had a collagen sponge with the bioactive agent BMP7, where each sponge had 1.5 mg BMP7 (Prospec Protein Specialists, Rehovot, Israel). Group 3 had no coating or bioactive agent. All animals retained normal function following surgery until sacrifice.

**TABLE 1. Experimental groups and time points (6,12 and 18 months) for the in vivo pilot PCL device cervical spine fusion study. Groups included CaP coated fusion device with no biologic, uncoated fusion device delivering BMP7 protein from a collagen sponge, and control with no coating or biologic.**

| Fusion Device Design | Surface Modification/Biologic | Number Animals/Time Point |
|---|---|---|
| Group 1: | CaP Coating | N=2; 6 months |
| PCL Cage, 5.3mm thick | No Biologic | N=1; 12 months |
| | | N=1; 18 months |
| Group 2: | No Coating | N=1; 6 months |
| PCL Cage, 5.3mm thick | BMP7 in Col Sponge | N=1; 12 months |
| | | N=1; 18 months |
| Group 3: | No Coating | N=2; 6 months |
| PCL Cage, 5.3mm thick | No Biologic | N=1; 12 months |
| | | N=1; 18 months |

The collagen sponges were prepared as follows. Seven mg bovine collagen type I (BD Biosciences), pH 7.4, was prepared in 2 ml PBS and cross-lined with 1 ml of 100 mM *N-*Ethyl-*N'*-(3-dimethylaminopropyl) carbodiimide hydrochloride (Sigma, St. Louis MO). After 15 minutes at 37 °C, a gel formed. Excess liquid was removed and the gel preparation was frozen by incubating on dry ice for 10 min. Lyophilization overnight followed. The lyophilized sponges were rinsed in distilled water to remove excess salt and rehydrate the sponges. After sterilizing with 70% ethanol, the sponges were re-lyophilized or air dried overnight. The sponges were then stored at 1-8 °C for up to 2 weeks until used.

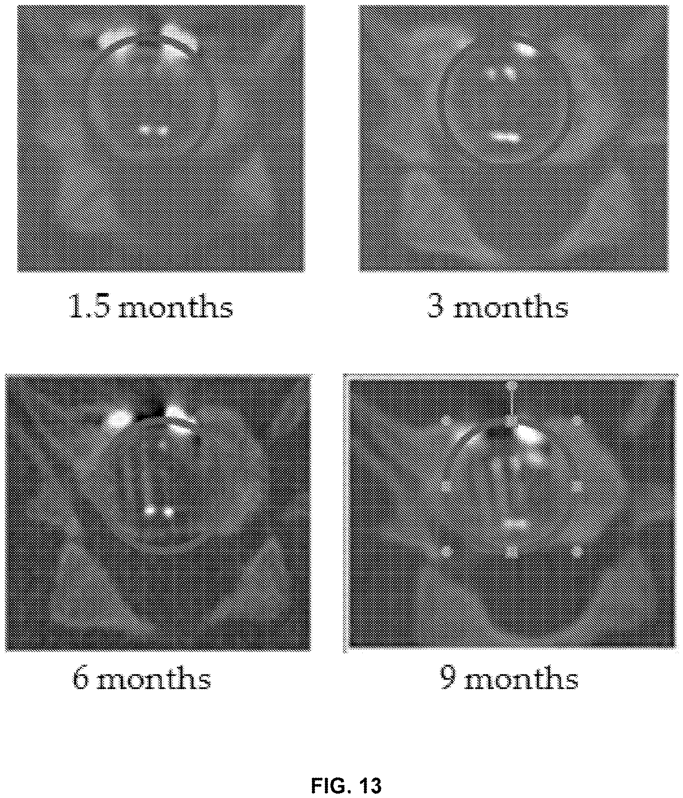

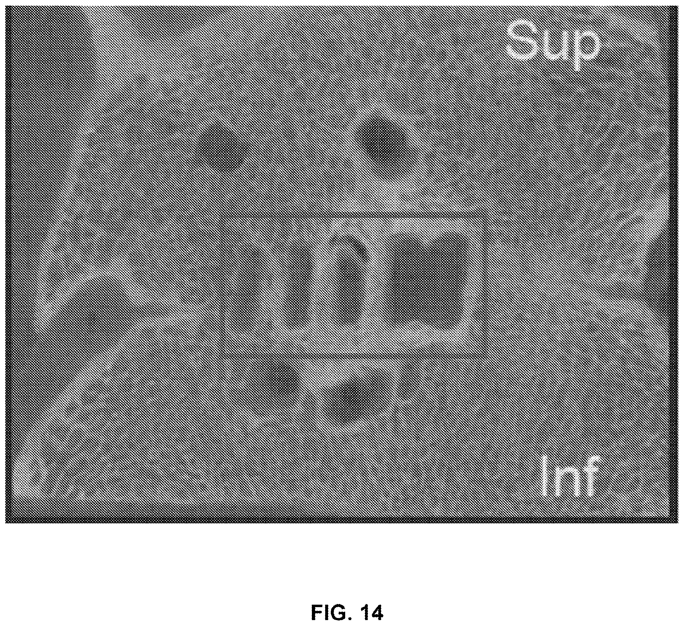

Bony fusion was assessed using Computed Tomography (CT) scans *in vivo* (FIG. 13) and micro-CT scans post-sacrifice (FIG. 14). All PCL fusion devices demonstrated bone ingrowth and both the CaP coated group (Exp Group 1, Table 1) and the BMP7 group (Exp Group 2, Table 1) demonstrated progression to bony bridging between 6 and 9 months (FIG. 13). Micro-CT scans provided higher resolution confirmation of these results (FIG. 14).

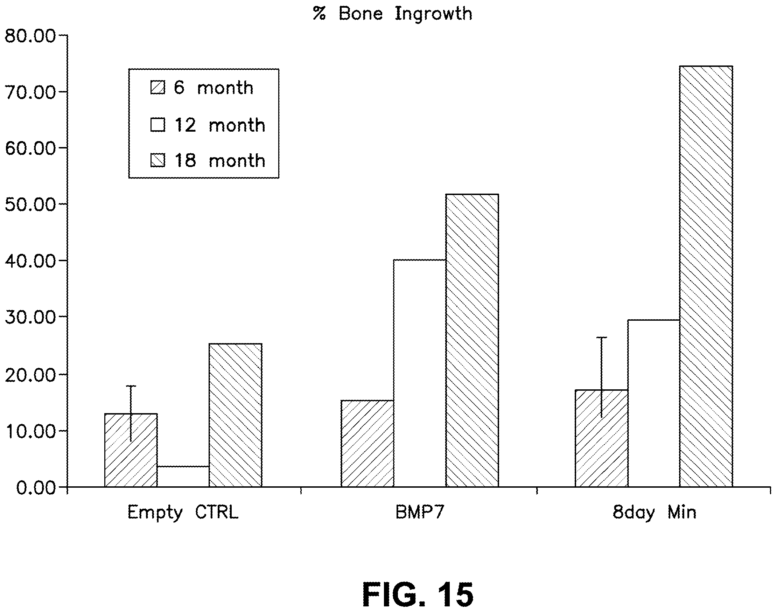

From the micro-CT scans, bone tissue was segmented and the percent of interbody pore volume filled by bone was calculated (100% bone volume fraction indicates complete bone filling of the interbody pores). The PCL/CaP coated implants with no biologic had as much or more bone fill than even the uncoated PCL cage delivering BMP7 from a sponge. Both groups had more bone fill than the uncoated PCL cage with no biologic (FIG. 15). All groups demonstrated increasing bone fill from 6 to 18 months, with the PCL/CaP coated fusion device (8day Min FIG. 15) showing 75% bone fill by 18 months.

In view of the above, it will be seen that the several advantages of the invention are achieved and other advantages attained.

As various changes could be made in the above methods and compositions without departing from the scope of the invention, it is intended that all matter contained in the above description and shown in the accompanying drawings shall be interpreted as illustrative and not in a limiting sense.

The discussion of the references herein is intended merely to summarize the assertions made by the authors and no admission is made that any reference constitutes prior art. Applicants reserve the right to challenge the accuracy and pertinence of the cited references.

## Claims

1. A cage (210) for facilitating fusion of adjacent bone segments, the cage comprising:
- a designed porous microstructure comprising a biocompatible material; and
- a fixation plate comprising a central portion (237),
**characterized in that**
the cage further comprises a plurality of substantially parallel spaced apart walls (212, 216, 221, 225, 229, 233), the walls interconnected by transverse projections;
the walls are substantially parallel to the central portion of the fixation plate and are coupled to the central portion of the fixation plate through at least one support (261), the support having a proximal end (262) and a distal end (263), the support being joined at the proximal end to the central portion (237) of the fixation plate, the support extending perpendicularly from the central portion (237).

2. The cage of claim 1, comprising at least one feature selected from the group consisting of:
(i) wherein the walls have a substantially rectangular transverse vertical cross section;
(ii) wherein the walls are coupled to the fixation plate through a base section that is substantially perpendicular to the central portion, and the fixation plate extends above and below the base section and walls and is slightly inwardly arcuate; or
(iii) wherein the walls have a substantially circular, oval, ovoid or polygonal transverse vertical cross section.

3. The cage of any one of claims 1-2, wherein the central portion (237) of the fixation plate comprises a midpoint and the cage comprises one support extending from the midpoint of the central portion, wherein the cage further comprises at least two flanges (264, 265) extending from the distal end of the support.

4. The cage of claim 3, wherein each flange (264, 265) extends in a direction such that the walls are positioned between the flanges and the central portion.

5. The cage of claim 4, wherein the flanges (264, 265) are substantially coextensive with the walls and the central portion.

6. The cage of claim 5, wherein the flanges (264, 265) are at least one of: substantially perpendicular to the support or coupled to form an inwardly directed arcuate member (266).

7. The cage of any one of claims 1-6, wherein the fixation plate further comprises a top section and a bottom section, wherein the top section of the fixation plate is above and in the same plane or a close parallel plane as the central portion of the fixation plate, and the bottom section of the fixation plate is below and in the same plane or a close parallel plane as the central portion of the fixation plate, wherein the top section of the fixation plate and the bottom section of the fixation plate each further comprise fastener holes extending therethrough suitable for accommodating fasteners to affix the cage to the adjacent bones.

8. The cage of any one of claims 1-7, designed to fit the parallel spaced apart walls into an intervertebral space between adjacent vertebrae, the intervertebral space being caused by the absence of an intervertebral disc.

9. The cage of any one of claims 1-8, wherein the biocompatible material is a polymeric material, a metallic material, a ceramic material, a degradable material or mixtures thereof.

10. The cage of claim 9, wherein the polymeric material is polycaprolactone, polylactide, polyglycolide, poly(lactide-glycolide), poly(propylene fumarate), poly(caprolactone fumarate), polyethylene glycol, poly(glycolide-co-caprolactone) or mixtures thereof.

11. The cage of any one of claims 1-10, further comprising an osteoconductive mineral coating on at least a portion of the designed porous microstructure selected from at least one of the following:
hydroxyapatite, a calcium-deficient carbonate-containing hydroxyapatite, a tricalcium phosphate, an amorphous calcium phosphate, an octacalcium phosphate, a dicalcium phosphate, a calcium phosphate, or a mixture thereof.

12. The cage of claim 11, wherein the osteoconductive mineral coating is calcium-deficient carbonate-containing hydroxyapatite.

13. The cage of claim 11 or 12, wherein the osteoconductive mineral coating substantially homogeneously covers the plurality of parallel spaced apart walls and transverse projections.

14. The cage of any one of claims 1-13, further comprising a bioactive agent, the bioactive agent being present in an amount that induces ossification between the adjacent bones, wherein the bioactive agent is selected from at least one of the following: a bone morphogenetic protein (BMP), demineralized bone matrix, a bone marrow aspirate, a transforming growth factor, a fibroblast growth factor, an insulin-like growth factor, a platelet derived growth factor, a vascular endothelial growth factor, a growth and development factor-5, platelet rich plasma, or a mixture thereof.

15. The cage of claim 14, wherein the bioactive agent is BMP2 or BMP7.

16. The cage of any one of claims 1-15, wherein
- the walls are substantially parallel to the central portion (237) and have a substantially rectangular transverse vertical cross section;
- the central portion (237) of the fixation plate comprises a midpoint and the support extends from the midpoint of the central portion, wherein the cage further comprises at least two flanges (264, 265) extending from the distal end of the support, wherein each flange extends in a direction such that the walls are positioned between the flanges and the central portion;
- the flanges (264, 265) are substantially coextensive with the walls and the central portion and substantially perpendicular to the support; and
- the flanges (264, 265) are coupled to form an inwardly directed arcuate member (266); the cage is designed to fit the parallel spaced apart walls into an intervertebral space created by the absence of an intervertebral disc between adjacent cervical vertebrae;
- the cage further comprises a top section of the fixation plate and a bottom section of the fixation plate, wherein the top section of the fixation plate is above and in the same plane or a close parallel plane as the central portion of the fixation plate, and the bottom section of the fixation plate is below and in the same plane or a close parallel plane as the central portion of the fixation plate, wherein the top section of the fixation plate and the bottom section of the fixation plate each further comprise fastener holes suitable for accommodating fasteners to affix the cage to the adjacent bones;
- the biocompatible material is polycaprolactone; and
- the cage further comprises a coating of calcium-deficient carbonate-containing hydroxyapatite that substantially homogeneously covers the plurality of parallel spaced apart walls and transverse projections.

17. The cage of any one of claims 1-16, fabricated using a solid free-form fabrication system.

## Patentansprüche

1. Käfig (210) zur erleichterten Fusion benachbarter Knochensegmente, wobei der Käfig umfasst:
- eine ausgebildete poröse Mikrostruktur, die ein biokompatibles Material umfasst; und
- eine Befestigungsplatte mit einem mittigen Abschnitt (237),
**dadurch gekennzeichnet, dass**
der Käfig zudem eine Vielzahl von im Wesentlichen parallel beabstandeten Wänden (212, 216, 221, 225, 229, 233) umfasst, wobei die Wände durch querverlaufende Vorsprünge miteinander verbunden sind;
die Wände im Wesentlichen parallel zum mittigen Abschnitt der Befestigungsplatte verlaufen und durch zumindest einen Träger (261) mit dem mittigen Abschnitt der Befestigungsplatte verbunden sind, wobei der Träger ein proximales Ende (262) und ein distales Ende (263) aufweist, der Träger am proximalen Ende mit dem mittigen Abschnitt (237) der Befestigungsplatte verbunden ist, wobei sich der Träger senkrecht vom mittigen Abschnitt (237) erstreckt.

2. Käfig nach Anspruch 1, umfassend zumindest ein Merkmal, ausgewählt aus der Gruppe bestehend aus:
(i) wobei die Wände einen im Wesentlichen rechteckigen querverlaufenden vertikalen Querschnitt aufweisen;
(ii) wobei die Wände mit der Befestigungsplatte durch einen Basisabschnitt verbunden sind, der im Wesentlichen senkrecht zum mittigen Abschnitt verläuft, und die Befestigungsplatte sich oberhalb und unterhalb des Basisabschnitts und der Wände erstreckt und leicht nach innen gebogen ist; oder
(iii) wobei die Wände einen im Wesentlichen kreisförmigen, ovalen, eiförmigen oder polygonalen querverlaufenden vertikalen Querschnitt aufweisen.

3. Käfig nach einem der Ansprüche 1 bis 2, wobei der mittige Abschnitt (237) der Befestigungsplatte einen Mittelpunkt umfasst, und der Käfig einen Träger umfasst, der sich vom Mittelpunkt des mittigen Abschnitts erstreckt, wobei der Käfig ferner zumindest zwei Ausleger (264, 265) umfasst, die sich vom distalen Ende des Trägers erstrecken.

4. Käfig nach Anspruch 3, wobei sich jeder Ausleger (264, 265) in eine Richtung erstreckt, sodass die Wände zwischen den Auslegern und dem mittigen Abschnitt positioniert sind.

5. Käfig nach Anspruch 4, wobei sich die Ausleger (264, 265) im Wesentlichen gemeinsam mit den Wänden und dem mittigen Abschnitt erstrecken.

6. Käfig nach Anspruch 5, wobei die Ausleger (264, 265) im Wesentlichen senkrecht zum Träger verlaufen, und/oder zum Bilden eines nach innen gerichteten bogenförmigen Elements (266) verbunden sind.

7. Käfig nach einem der Ansprüche 1 bis 6, wobei die Befestigungsplatte ferner einen oberen Abschnitt und einen unteren Abschnitt aufweist, wobei der obere Abschnitt der Befestigungsplatte oberhalb und in derselben Ebene oder in einer nahen parallelen Ebene wie der mittlere Abschnitt der Befestigungsplatte liegt, und der untere Abschnitt der Befestigungsplatte unterhalb und in derselben Ebene oder in einer nahen parallelen Ebene wie der mittlere Abschnitt der Befestigungsplatte liegt, wobei der obere Abschnitt der Befestigungsplatte und der untere Abschnitt der Befestigungsplatte zudem jeweils Befestigungsöffnungen umfassen, die sich durch diese hindurch erstrecken, die zur Aufnahme von Befestigungselementen geeignet sind, um den Käfig an den benachbarten Knochen anzubringen.

8. Käfig nach einem der Ansprüche 1 bis 7, der zum Einpassen der parallel beabstandeten Wände in einen Wirbelzwischenraum zwischen benachbarten Wirbeln ausgelegt ist, wobei der Wirbelzwischenraum durch das Fehlen einer Bandscheibe verursacht wird.

9. Käfig nach einem der Ansprüche 1 bis 8, wobei das biokompatible Material ein Polymermaterial, ein Metallmaterial, ein Keramikmaterial, ein abbaubares Material oder Mischungen davon ist.

10. Käfig nach Anspruch 9, wobei das Polymermaterial Polycaprolacton, Polylactid, Polyglycolid, Poly(lactid-glycolid), Poly(propylenfumarat), Poly(caprolactonfumarat), Polyethylenglycol, Poly(glycolid-co-caprolacton) oder Mischungen davon ist.

11. Käfig nach einem der Ansprüche 1 bis 10, ferner umfassend eine osteokonduktive Mineralbeschichtung auf zumindest einem Teil der ausgebildeten porösen Mikrostruktur, die aus Folgenden ausgewählt ist:
Hydroxylapatit, Calcium-defizitärem carbonathaltigen Hydroxylapatit, Tricalciumphosphat, amorphem Calciumphosphat, Octacalciumphosphat, Dicalciumphosphat, Calciumphosphat und/oder einer Mischung davon.

12. Käfig nach Anspruch 11, wobei die osteokonduktive Mineralbeschichtung Calciumdefizitärer carbonathaltiger Hydroxylapatit ist.

13. Käfig nach Anspruch 11 oder 12, wobei die osteokonduktive Mineralbeschichtung die Vielzahl der parallel beabstandeten Wände und querverlaufenden Vorsprünge im Wesentlichen homogen bedeckt.

14. Käfig nach einem der Ansprüche 1 bis 13, ferner umfassend ein bioaktives Mittel, wobei das bioaktive Mittel in einer Menge vorliegt, die eine Verknöcherung zwischen den benachbarten Knochen induziert, wobei das bioaktive Mittel aus Folgenden ausgewählt ist: einem knochenmorphogenetischen Protein (BMP), einer demineralisierten Knochenmatrix, einem Knochenmarksaspirat, einem transformierenden Wachstumsfaktor, einem Fibroblastenwachstumsfaktor, einem insulinartigen Wachstumsfaktor, einem von Thrombozyten abstammenden Wachstumsfaktor, einem vaskulären endothelialen Wachstumsfaktor, einem Wachstums- und Entwicklungsfaktor -5, thrombozytenreichem Plasma, und/oder einer Mischung davon.

15. Käfig nach Anspruch 14, wobei das bioaktive Mittel BMP2 oder BMP7 ist.

16. Käfig nach einem der Ansprüche 1 bis 15, wobei
- die Wände im Wesentlichen parallel zum mittigen Abschnitt (237) verlaufen und einen im Wesentlichen rechteckigen querverlaufenden vertikalen Querschnitt aufweisen;
- der mittige Abschnitt (237) der Befestigungsplatte einen Mittelpunkt aufweist und der Träger sich vom Mittelpunkt des mittigen Abschnitts erstreckt, wobei der Käfig ferner zumindest zwei Ausleger (264, 265) umfasst, die sich vom distalen Ende des Trägers erstrecken, wobei sich jeder Ausleger in einer Richtung erstreckt, sodass die Wände zwischen den Auslegern und dem mittigen Abschnitt positioniert sind;
- die Ausleger (264, 265) sich im Wesentlichen gemeinsam mit den Wänden und dem mittigen Abschnitt erstrecken und im Wesentlichen senkrecht zum Träger verlaufen; und
- die Ausleger (264, 265) zum Bilden eines nach innen gerichteten bogenförmiges Elements (266) verbunden sind; wobei der Käfig zum Einpassen der parallel beabstandeten Wände in einen Wirbelzwischenraum ausgelegt ist, der durch das Fehlen einer Bandscheibe zwischen benachbarten Halswirbeln verursacht wird;
- der Käfig ferner einen oberen Abschnitt der Befestigungsplatte und einen unteren Abschnitt der Befestigungsplatte aufweist, wobei der obere Abschnitt der Befestigungsplatte oberhalb und in derselben Ebene oder in einer nahen parallelen Ebene wie der mittlere Abschnitt der Befestigungsplatte liegt, und der untere Abschnitt der Befestigungsplatte unterhalb und in derselben Ebene oder in einer nahen parallelen Ebene wie der mittlere Abschnitt der Befestigungsplatte liegt, wobei der obere Abschnitt der Befestigungsplatte und der untere Abschnitt der Befestigungsplatte zudem jeweils Befestigungsöffnungen umfassen, die zur Aufnahme von Befestigungselementen geeignet sind, um den Käfig an den benachbarten Knochen zu befestigen;
- das biokompatible Material ist Polycaprolacton; und
- der Käfig ferner eine Beschichtung aus Calcium-defizitärem carbonathaltigem Hydroxylapatit umfasst, welche die Vielzahl der parallel beabstandeten Wände und querverlaufenden Vorsprünge im Wesentlichen homogen bedeckt.

17. Käfig nach einem der Ansprüche 1 bis 16, der unter Verwendung eines festen Freiformherstellungssystems hergestellt ist.

## Revendications

1. Cage (210) destinée à faciliter la fusion de segments osseux adjacents, la cage comprenant :
- une microstructure de conception poreuse comprenant un matériau biocompatible, et
- une plaque de fixation comprenant une portion centrale (237),
**caractérisée en ce que**
la cage comprend en outre une pluralité de parois espacées les unes des autres et en grande partie parallèles (212, 216, 221, 225, 229, 233), les parois étant reliées entre elles par des saillies transversales ;
les parois sont en grande partie parallèles à la portion centrale de la plaque de fixation et sont couplées à la portion centrale de la plaque de fixation par le biais d'au moins un support (261), le support présentant une extrémité proximale (262) et une extrémité distale (263), le support étant joint sur l'extrémité proximale à la portion centrale (237) de la plaque de fixation, le support s'étendant perpendiculairement à la portion centrale (237).

2. Cage selon la revendication 1, comprenant en outre au moins une caractéristique sélectionnée parmi le groupe suivant :
(i) dans laquelle les parois présentent une section en coupe verticale transversale en grande partie rectangulaire ;
(ii) dans laquelle les parois sont couplées à la plaque de fixation par le biais d'une section de base, laquelle est en grande partie perpendiculaire à la portion centrale, et la plaque de fixation s'étend au-dessus et au-dessous de la section de base et des parois, et est légèrement arquée vers l'intérieur, et
(iii) dans laquelle les parois présentent une section en coupe verticale transversale en grande partie circulaire, ovale, ovoïde ou polygonale.

3. Cage selon l'une quelconque des revendications 1 à 2, dans laquelle la portion centrale (237) de la plaque de fixation comprend un point de milieu, et la cage comprend un support s'étendant à partir du point de milieu de la portion centrale, et dans laquelle la cage comprend en outre au moins deux rebords (264, 265) s'étendant à partir de l'extrémité distale du support.

4. Cage selon la revendication 3, dans laquelle chaque rebord (264, 265) s'étend dans une direction telle que les parois sont positionnées entre les rebords et la portion centrale.

5. Cage selon la revendication 4, dans laquelle les rebords (264, 265) sont en grande partie en co-extension avec les parois et la portion centrale.

6. Cage selon la revendication 5, dans laquelle les rebords (264, 265) sont au moins : en grande partie perpendiculaires au support ou couplés pour former un élément arqué dirigé vers l'intérieur (266).

7. Cage selon l'une quelconque des revendications 1 à 6, dans laquelle la plaque de fixation comprend en outre une section supérieure et une section inférieure ; dans laquelle la section supérieure de la plaque de fixation est au-dessus de la portion centrale de la plaque de fixation et dans le même plan que celle-ci, ou dans un plan parallèle proche de celle-ci, et la section inférieure de la plaque de fixation est au-dessous de la portion centrale de la plaque de fixation, et dans le même plan que celle-ci, ou dans un plan parallèle proche de celle-ci ; dans laquelle la section supérieure de la plaque de fixation et la section inférieure de la plaque de fixation comprennent en outre des trous d'attache s'étendant à travers elles et convenant pour loger des attaches pour fixer la cage sur les os adjacents.

8. Cage selon l'une quelconque des revendications 1 à 7, conçue pour installer les parois espacées entre elles et parallèles dans un espace intervertébral entre des vertèbres adjacentes, l'espace intervertébral étant provoqué par l'absence d'un disque intervertébral.

9. Cage selon l'une quelconque des revendications 1 à 8, dans laquelle le matériau biocompatible est un matériau polymère, un matériau métallique, un matériau céramique, un matériau dégradable, ou des mélanges de ceux-ci.

10. Cage selon la revendication 9, dans laquelle le matériau polymère est une polycaprolactone, un polylactide, un polyglycolide, un poly(lactide-glycolide), un poly(propylène fumarate), un poly(caprolactone fumarate), un polyéthylène glycol, une poly(glycolide-co-caprolactone), ou des mélanges de ceux-ci.

11. Cage selon l'une quelconque des revendications 1 à 10, comprenant en outre un revêtement minéral ostéoconducteur sur au moins une portion de la microstructure de conception poreuse, sélectionné parmi au moins un des éléments suivants :
une hydroxyapatite, une hydroxyapatite pauvre en calcium contenant du carbonate, un phosphate tricalcique, un phosphate de calcium amorphe, un phosphate d'octacalcium, un phosphate dicalcique, un phosphate de calcium, ou un mélange de ceux-ci.

12. Cage selon la revendication 11, dans laquelle le revêtement minéral ostéoconducteur est une hydroxyapatite pauvre en calcium contenant du carbonate.

13. Cage selon la revendication 11 ou 12, dans laquelle le revêtement minéral ostéoconducteur recouvre de manière en grande partie homogène la pluralité de parois espacées entre elles et parallèles et des saillies transversales.

14. Cage selon l'une quelconque des revendications 1 à 13, comprenant en outre un agent bioactif, l'agent bioactif étant présent en une quantité qui induit une ossification entre les os adjacents, dans laquelle l'agent bioactif est sélectionné parmi au moins un des éléments suivants : une protéine morphogénétique osseuse (BMP, bone morphogenetic protein), une matrice osseuse déminéralisée, un aspirat de moelle osseuse, un facteur de croissance transformant, un facteur de croissance de fibroblastes, un facteur de croissance insulinomimétique, un facteur de croissance dérivé de plaquettes, un facteur de croissance endothéliale vasculaire, un facteur de développement et de croissance de type 5, du plasma riche en plaquettes, ou un mélange de ceux-ci.

15. Cage selon la revendication 14, dans laquelle l'agent bioactif est BMP2 ou BMP7.

16. Cage selon l'une quelconque des revendications 1 à 15, dans laquelle
- les parois sont en grande partie parallèles à la portion centrale (237) et présentent une section en coupe verticale transversale en grande partie rectangulaire ;
- la portion centrale (237) de la plaque de fixation comprend un point de milieu, et le support s'étend à partir du point de milieu de la portion centrale, dans laquelle la cage comprend en outre au moins deux rebords (264, 265) s'étendant à partir de l'extrémité distale du support ; dans laquelle chaque rebord s'étend dans une direction telle que les parois sont positionnées entre les rebords et la portion centrale ;
- les rebords (264, 265) sont en grande partie en co-extension avec les parois et la portion centrale et en grande partie perpendiculaires au support, et
- les rebords (264), 265) sont couplés pour former un élément arqué dirigé vers l'intérieur (266) ; la cage est conçue pour installer les parois espacées entre elles et parallèles dans un espace intervertébral créé par l'absence d'un disque intervertébral entre des vertèbres cervicales adjacentes ;
- la cage comprend en outre une section supérieure de la plaque de fixation et une section inférieure de la plaque de fixation ; dans laquelle la section supérieure de la plaque de fixation est au-dessus de la portion centrale de la plaque de fixation et dans le même plan que celle-ci, ou dans un plan parallèle proche de celle-ci ; et la section inférieure de la plaque de fixation est au-dessous de la portion centrale de la plaque de fixation et dans le même plan que celle-ci, ou dans un plan parallèle proche de celle-ci ; dans laquelle la section supérieure de la plaque de fixation et la section inférieure de la plaque de fixation comprennent en outre des trous d'attache convenant pour loger des attaches pour fixer la cage sur les os adjacents ;
- le matériau biocompatible est une polycaprolactone, et
- la cage comprend en outre un revêtement d'hydroxyapatite pauvre en calcium contenant du carbonate, lequel recouvre de manière en grande partie homogène la pluralité de parois espacées parallèles et des saillies transversales.

17. Cage selon l'une quelconque des revendications 1 à 16, fabriquée à l'aide d'un système de fabrication de solide de forme libre.
